# EUROPEAN PATENT SPECIFICATION

(11) **EP 3 478 717 B1**
(45) Date of publication and mention of the grant of the patent: **05.01.2022**
(21) Application number: 17737762.9
(22) Date of filing: 03.07.2017
(51) Int. Cl.: C07K 16/22

(54) **NOVEL ANTIBODY FORMAT**
NEUARTIGES ANTIKÖRPERFORMAT
NOUVEAU FORMAT D'ANTICORPS

(30) Priority: 04.07.2016 EP 16177746
(43) Date of publication of application: 08.05.2019
(73) Proprietor: F. Hoffmann-La Roche AG, 4070 Basel (CH)
(72) Inventor: DENGL, Stefan, 82377 Penzberg (DE); HUELSMANN, Peter Michael, 82392 Habach (DE)
(74) Representative: Skolaut, Alexander
(86) International application number: PCT/EP2017/066495
(87) International publication number: WO 2018/007314

(56) References cited:
- WO-A1-2009/018386
- WO-A1-2010/040508
- WO-A1-2012/069466
- WO-A1-2014/009465
- WO-A1-2016/087416
- SEBASTIAN FENN ET AL: "Crystal Structure of an Anti-Ang2 CrossFab Demonstrates Complete Structural and Functional Integrity of the Variable Domain", PLOS ONE, vol. 8, no. 4, 1 April 2013 (2013-04-01), page e61953, XP55106055, ISSN: 1932-6203, DOI: 10.1371/journal.pone.0061953
- J?rg T Regula ET AL: "Targeting key angiogenic pathways with a bispecific CrossMAb optimized for neovascular eye diseases", EMBO Molecular Medicine, 1 November 2016 (2016-11-01), pages 1265-1288, XP055404923, Frankfurt DOI: 10.15252/emmm.201505889 Retrieved from the Internet: URL:http://onlinelibrary.wiley.com/store/1 0.15252/emmm.201505889/asset/emmm201505889 .pdf?v=1&t=j7bif84l&s=9281f41f2bc631e3568b 4600c36b304609f6fac3
- Margitta Alt ET AL: "Novel tetravalent and bispeci?c IgG-like antibody molecules combining single-chain diabodies with the immunoglobulin Q1 Fc or CH3 region", , 1 July 1999 (1999-07-01), XP055405061, Retrieved from the Internet: URL:http://onlinelibrary.wiley.com/store/1 0.1016/S0014-5793(99)00782-6/asset/feb2s00 14579399007826.pdf?v=1&t=j7bsdwab&s=48245c 84177e748c464c3662262461628f11978e [retrieved on 2017-09-08]

### SEQUENCE LISTING

<110> F. Hoffmann-La Roche AG
<120> Novel antibody format
<130> P32965-WO
<150> EP16177746.1
   <151> 2016-07-04
<160> 25
<170> PatentIn version 3.5
<210> 1
   <211> 11
   <212> PRT
   <213> Artificial Sequence
<220>
   <223> linker-1
<400> 1
   Thr Val Ser Ser Gly Gly Gly Pro Arg Glu Pro
   1               5                   10
<210> 2
   <211> 7
   <212> PRT
   <213> Artificial Sequence
<220>
   <223> linker-2
<400> 2
   Leu Ser Leu Ser Gly Gly Gly
   1 5
<210> 3
   <211> 13
   <212> PRT
   <213> Artificial Sequence
<220>
   <223> linker-3
<400> 3
   Thr Val Ser Ser Gly Gly Gly Gly Ser Pro Arg Glu Pro
   1 5 10
<210> 4
   <211> 9
   <212> PRT
   <213> Artificial Sequence
<220>
   <223> linker-4
<400> 4
   Leu Ser Leu Ser Gly Gly Gly Gly Ser
   1 5
<210> 5
   <211> 11
   <212> PRT
   <213> Artificial Sequence
<220>
   <223> linker-5
<400> 5
   Thr Val Ser Ser Gly Pro Gly Pro Arg Glu Pro
   1 5 10
<210> 6
   <211> 7
   <212> PRT
   <213> Artificial Sequence
<220>
   <223> linker-6
<400> 6
   Leu Ser Leu Ser Gly Pro Gly
   1 5
<210> 7
   <211> 11
   <212> PRT
   <213> Artificial Sequence
<220>
   <223> linker-7
<400> 7
   Thr Val Ser Ser Gly Pro Pro Ser Arg Glu Pro
   1               5                   10
<210> 8
   <211> 7
   <212> PRT
   <213> Artificial Sequence
<220>
   <223> linker-8
<400> 8
   Leu Ser Leu Ser Pro Pro Gly
   1               5
<210> 9
   <211> 6
   <212> PRT
   <213> Artificial Sequence
<220>
   <223> C-terminal cap-1
<400> 9
   Gly Ser Glu Pro Glu Ala
   1               5
<210> 10
   <211> 107
   <212> PRT
   <213> Artificial Sequence
<220>
   <223> CH3-hole
<400> 10
   Gly Gln Pro Arg Glu Pro Gln Val Cys Thr Leu Pro Pro Ser Arg Asp
   1               5                   10                  15
   Glu Leu Thr Lys Asn Gln Val Ser Leu Ser Cys Ala Val Lys Gly Phe
               20                  25                  30
   Tyr Pro Ser Asp Ile Ala Val Glu Trp Glu Ser Asn Gly Gln Pro Glu
           35                  40                  45
   Asn Asn Tyr Lys Thr Thr Pro Pro Val Leu Asp Ser Asp Gly Ser Phe
       50                  55                  60
   Phe Leu Val Ser Lys Leu Thr Val Asp Lys Ser Arg Trp Gln Gln Gly
   65                  70                  75                  80
   Asn Val Phe Ser Cys Ser Val Met His Glu Ala Leu His Asn His Tyr
                   85                  90                  95
   Thr Gln Lys Ser Leu Ser Leu Ser Pro Gly Lys
               100                 105
<210> 11
   <211> 107
   <212> PRT
   <213> Artificial Sequence
<220>
   <223> CH3-knob
<400> 11
   Gly Gln Pro Arg Glu Pro Gln Val Tyr Thr Leu Pro Pro Cys Arg Asp
   1               5                   10                  15
   Glu Leu Thr Lys Asn Gln Val Ser Leu Trp Cys Leu Val Lys Gly Phe
               20                  25                  30
   Tyr Pro Ser Asp Ile Ala Val Glu Trp Glu Ser Asn Gly Gln Pro Glu
           35                  40                  45
   Asn Asn Tyr Lys Thr Thr Pro Pro Val Leu Asp Ser Asp Gly Ser Phe
       50                  55                  60
   Phe Leu Tyr Ser Lys Leu Thr Val Asp Lys Ser Arg Trp Gln Gln Gly
   65                  70                  75                  80
   Asn Val Phe Ser Cys Ser Val Met His Glu Ala Leu His Asn His Tyr
                   85                  90                  95
   Thr Gln Lys Ser Leu Ser Leu Ser Pro Gly Lys
               100                 105
<210> 12
   <211> 107
   <212> PRT
   <213> Artificial Sequence
<220>
   <223> CH3-hole-C
<400> 12
   Gly Gln Pro Arg Glu Pro Gln Val Cys Thr Leu Pro Pro Ser Arg Asp
   1               5                   10                  15
   Glu Leu Thr Lys Asn Gln Val Ser Leu Ser Cys Ala Val Lys Gly Phe
               20                  25                  30
   Tyr Pro Ser Asp Ile Ala Val Glu Trp Glu Ser Asn Gly Gln Pro Glu
           35                  40                  45
   Asn Asn Tyr Lys Thr Thr Pro Pro Val Leu Asp Ser Asp Gly Ser Phe
       50                  55                  60
   Phe Leu Val Ser Lys Leu Thr Val Asp Lys Ser Arg Trp Gln Gln Gly
   65                  70                  75                  80
   Asn Val Phe Ser Cys Ser Val Met His Glu Ala Leu Cys Asn His Tyr
                   85                  90                  95
   Thr Gln Lys Ser Leu Ser Leu Ser Pro Gly Lys
                       100                 105
<210> 13
   <211> 107
   <212> PRT
   <213> Artificial Sequence
<220>
   <223> CH3-knob-C
<400> 13
   Gly Gln Pro Arg Glu Pro Gln Val Tyr Thr Leu Pro Pro Cys Arg Asp
   1 5 10 15
   Glu Leu Thr Lys Asn Gln Val Ser Leu Trp Cys Leu Val Lys Gly Phe
   20 25 30
   Tyr Pro Ser Asp Ile Ala Val Glu Trp Glu Ser Asn Gly Gln Pro Glu
   35 40 45
   Asn Asn Tyr Lys Thr Thr Pro Pro Val Leu Asp Ser Asp Gly Ser Phe
   50 55 60
   Phe Leu Tyr Ser Lys Leu Thr Val Asp Lys Ser Arg Trp Gln Gln Gly
   65 70 75 80
   Asn Val Phe Ser Cys Ser Val Met His Glu Ala Leu Cys Asn His Tyr
   85 90 95
   Thr Gln Lys Ser Leu Ser Leu Ser Pro Gly Lys
   100 105
<210> 14
   <211> 123
   <212> PRT
   <213> Artificial
<220>
   <223> heavy chain variable domain VH, <VEGF>ranibizumab
<400> 14
<210> 15
   <211> 123
   <212> PRT
   <213> Artificial Sequence
<220>
   <223> heavy chain variable domain VH, <VEGF>ranibizumab, C-variant
<400> 15
   Glu Val Gln Leu Val Glu Ser Gly Gly Gly Leu Val Gln Pro Gly Gly
   1 5 10 15
   Ser Leu Arg Leu Ser Cys Ala Ala Ser Gly Tyr Asp Phe Thr His Tyr
   20 25 30
   Gly Met Asn Trp Val Arg Gln Ala Pro Gly Lys Gly Leu Glu Trp Val
   35 40 45
   Gly Trp Ile Asn Thr Tyr Thr Gly Glu Pro Thr Tyr Ala Ala Asp Phe
   50 55 60
   Lys Arg Arg Phe Thr Phe Ser Leu Asp Thr Ser Lys Ser Thr Ala Tyr
   65 70 75 80
   Leu Gln Met Asn Cys Leu Arg Ala Glu Asp Thr Ala Val Tyr Tyr Cys
   85 90 95
   Ala Lys Tyr Pro Tyr Tyr Tyr Gly Thr Ser His Trp Tyr Phe Asp Val
   100 105 110
   Trp Gly Gln Gly Thr Leu Val Thr Val Ser Ser
   115 120
<210> 16
   <211> 107
   <212> PRT
   <213> Artificial
<220>
   <223> light chain variable domain VL, <VEGF>ranibizumab
<400> 16
<210> 17
   <211> 129
   <212> PRT
   <213> Artificial
<220>
   <223> heavy chain variable domain VH, <ANG-2> Ang2
<400> 17
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Gly Tyr
   20 25 30
   Tyr Met His Trp Val Arg Gln Ala Pro Gly Gln Gly Leu Glu Trp Met
   35 40 45
   Gly Trp Ile Asn Pro Asn Ser Gly Gly Thr Asn Tyr Ala Gln Lys Phe
   50 55 60
   Gln Gly Arg Val Thr Met Thr Arg Asp Thr Ser Ile Ser Thr Ala Tyr
   65 70 75 80
   Met Glu Leu Ser Arg Leu Arg Ser Asp Asp Thr Ala Val Tyr Tyr Cys
   85 90 95
   Ala Arg Ser Pro Asn Pro Tyr Tyr Tyr Asp Ser Ser Gly Tyr Tyr Tyr
   100 105 110
   Pro Gly Ala Phe Asp Ile Trp Gly Gln Gly Thr Met Val Thr Val Ser
   115 120 125
   Ser
<210> 18
   <211> 129
   <212> PRT
   <213> Artificial Sequence
<220>
   <223> heavy chain variable domain VH, <ANG-2> Ang2, C-variant
<400> 18
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Gly Tyr
   20 25 30
   Tyr Met His Trp Val Arg Gln Ala Pro Gly Gln Gly Leu Glu Trp Met
   35 40 45
   Gly Trp Ile Asn Pro Asn Ser Gly Gly Thr Asn Tyr Ala Gln Lys Phe
   50 55 60
   Gln Gly Arg Val Thr Met Thr Arg Asp Thr Ser Ile Ser Thr Ala Tyr
   65 70 75 80
   Met Glu Leu Ser Cys Leu Arg Ser Asp Asp Thr Ala Val Tyr Tyr Cys
   85 90 95
   Ala Arg Ser Pro Asn Pro Tyr Tyr Tyr Asp Ser Ser Gly Tyr Tyr Tyr
   100 105 110
   Pro Gly Ala Phe Asp Ile Trp Gly Gln Gly Thr Met Val Thr Val Ser
   115 120 125
   Ser
<210> 19
   <211> 108
   <212> PRT
   <213> Artificial
<220>
   <223> light chain variable domain VL, <ANG-2> Ang2
<400> 19
   Ser Tyr Val Leu Thr Gln Pro Pro Ser Val Ser Val Ala Pro Gly Gln
   1               5                   10                  15
   Thr Ala Arg Ile Thr Cys Gly Gly Asn Asn Ile Gly Ser Lys Ser Val
               20                  25                  30
   His Trp Tyr Gln Gln Lys Pro Gly Gln Ala Pro Val Leu Val Val Tyr
           35                  40                  45
   Asp Asp Ser Asp Arg Pro Ser Gly Ile Pro Glu Arg Phe Ser Gly Ser
       50                  55                  60
   Asn Ser Gly Asn Thr Ala Thr Leu Thr Ile Ser Arg Val Glu Ala Gly
   65                  70                  75                  80
   Asp Glu Ala Asp Tyr Tyr Cys Gln Val Trp Asp Ser Ser Ser Asp His
                   85                  90                  95
   Trp Val Phe Gly Gly Gly Thr Lys Leu Thr Val Leu
               100                 105
<210> 20
   <211> 6
   <212> PRT
   <213> Artificial Sequence
<220>
   <223> C-terminal cap-2
<400> 20
   Ala Ser Glu Pro Glu Ala
   1               5
<210> 21
   <211> 107
   <212> PRT
   <213> Homo sapiens
<400> 21
<210> 22
   <211> 105
   <212> PRT
   <213> Homo sapiens
<400> 22
   Gln Pro Lys Ala Ala Pro Ser Val Thr Leu Phe Pro Pro Ser Ser Glu
   1               5                   10                  15
   Glu Leu Gln Ala Asn Lys Ala Thr Leu Val Cys Leu Ile Ser Asp Phe
               20                  25                  30
   Tyr Pro Gly Ala Val Thr Val Ala Trp Lys Ala Asp Ser Ser Pro Val
           35                  40                  45
   Lys Ala Gly Val Glu Thr Thr Thr Pro Ser Lys Gln Ser Asn Asn Lys
       50                  55                  60
   Tyr Ala Ala Ser Ser Tyr Leu Ser Leu Thr Pro Glu Gln Trp Lys Ser
   65                  70                  75                  80
   His Arg Ser Tyr Ser Cys Gln Val Thr His Glu Gly Ser Thr Val Glu
                   85                  90                  95
   Lys Thr Val Ala Pro Thr Glu Cys Ser
               100                 105
<210> 23
   <211> 107
   <212> PRT
   <213> Homo sapiens
<400> 23
   Ala Pro Glu Leu Leu Gly Gly Pro Ser Val Phe Leu Phe Pro Pro Lys
   1               5                   10                  15
   Pro Lys Asp Thr Leu Met Ile Ser Arg Thr Pro Glu Val Thr Cys Val
               20                  25                  30
   Trp Asp Val Ser His Glu Asp Pro Glu Val Lys Phe Asn Trp Tyr Val
           35                  40                  45
   Asp Gly Val Glu Val His Asn Ala Lys Thr Lys Pro Arg Glu Glu Gln
       50                  55                  60
   Glu Ser Thr Tyr Arg Trp Ser Val Leu Thr Val Leu His Gln Asp Trp
   65                  70                  75                  80
   Leu Asn Gly Lys Glu Tyr Lys Cys Lys Val Ser Asn Lys Ala Leu Pro
                   85                  90                  95
   Ala Pro Ile Glu Lys Thr Ile Ser Lys Ala Lys
               100                 105
<210> 24
   <211> 105
   <212> **PRT**
   <213> Homo sapiens
<400> 24
   Gly Gln Pro Arg Glu Pro Gln Val Tyr Thr Leu Pro Pro Ser Arg Asp
   1               5                   10                  15
   Glu Leu Thr Lys Asn Gln Val Ser Leu Thr Cys Leu Val Lys Gly Phe
               20                  25                  30
   Tyr Pro Ser Asp Ile Ala Val Glu Trp Glu Ser Asn Gly Gln Pro Glu
           35                  40                  45
   Asn Asn Tyr Lys Thr Thr Pro Pro Val Leu Asp Ser Asp Gly Ser Phe
       50                  55                  60
   Phe Leu Tyr Ser Lys Leu Thr Val Asp Lys Ser Arg Trp Gln Gln Gly
   65                  70                  75                  80
   Asn Val Phe Ser Cys Ser Val Met His Glu Ala Leu His Asn His Tyr
                   85                  90                  95
   Thr Gln Lys Ser Leu Ser Leu Ser Pro
               100                 105
<210> 25
   <211> 98
   <212> PRT
   <213> Homo sapiens
<400> 25
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val

## Description

A novel antibody format having, amongst other things, a reduced molecular weight in comparison to a full-length antibody and comprising two antigen binding sites as well as uses thereof are described herein.

### Technical Background

Age-related macular degeneration is the most common eye disease, causing irreversible blindness in persons older than 50 years of age in industrialized countries. Present therapies are based, for example, on VEGF inhibition by antibodies/antibody formats, and require repeated intravitreal injection at intervals of 4 to 6 weeks. Furthermore, some patients do not respond to the available anti-VEGF monotherapies. Intensive research is being conducted on combination therapies based on already proven anti-VEGF medicaments and/or novel target molecules, and having the objective of increasing the treatment efficiency and safety and reducing the frequency of injections.

One of the factors driving the clearance from drug from the eye is diffusion. The diffusive properties of a drug are mainly determined by the size of the drug eventually in combination with Fc-receptor binding.

After clearance from the eye the drug can be found in the systemic circulation. Thus, high dose application and concomitant high drug clearance from eye results in systemic drug exposure, which increases risk of potential side effects.

New antibody formats derived from the wild-type four chain Y-shaped antibody format have been developed in the last years. These formats are mainly bi- and multispecific formats. For a review see e.g. Kontermann, R., mAbs 4 (2012) 182-197.

For example, in US 2009/0175867 a single-chain multivalent binding protein with effector function is disclosed. In WO 2014/131711 bispecific antibodies are disclosed wherein the second and the third antigen binding moiety may be fused to the Fc domain directly or through an immunoglobulin hinge region. In WO 2011/117329 bispecific, bivalent anti-VEGF/ANG-2 antibodies are disclosed. WO 2009/080253 discloses bivalent, bispecific antibodies. WO2016/087416 discloses the use of a CH3 domain as a dimerization unit.

### Summary of the Invention

Herein is reported a novel, two chain, low molecular weight, bivalent antibody, which can serve as a basis for producing novel therapeutic agents. Such an antibody can be used, for example, in ophthalmological diseases, such as, e.g., age-related macular degeneration, as it has improved properties for ophthalmologic applications. Likewise this format can have applications in oncologic, neurological (including crossing the blood-brain-barrier) and anti-inflammatory diseases.

It has been found that, amongst other things, the introduction of an intra-chain disulfide bridge increases the stability of the novel bivalent antibody as reported herein. In more detail, the introduction of an intrachain disulfide bond between one of the antibody variable domains and the antibody constant domain resulted in an improvement of the structural stability of this new antibody (format), as evidenced e.g. by the increase in the melting temperature of the antibody.

The scope of the invention disclosed herein is defined by the claims.

One aspect as reported herein is a (recombinant) fusion polypeptide comprising an antibody heavy chain variable domain, an antibody CH3 domain as multimerization domain and an antibody light chain variable domain,
wherein
   - the antibody heavy chain variable domain is fused via a first (peptidic) linker to one terminus of the multimerization domain,
   - the antibody light chain variable domain is fused via a second (peptidic) linker to the respective other terminus of the multimerization domain, and
   - the antibody heavy chain variable domain has a intra-peptidic disulphide bond to the antibody CH3 multimerization domain,
wherein the heavy chain variable domain comprises at position 82b according to Kabat a cysteine amino acid residue, wherein the antibody CH3 domain has at position 433 according to Kabat a cysteine amino acid residue,
wherein the disulfide bind is between the cysteine residue at position 82b of the heavy chain variable domain and the cysteine residue at position 433 of the CH3 domain, and
wherein the N-terminal and C-terminal domains of the fusion polypeptide form a binding site.

In one embodiment the polypeptide or the fusion polypeptide comprises in N- to C-terminal direction an antibody heavy chain variable domain, a multimerization domain and an antibody light chain variable domain.

In one embodiment the polypeptide or the fusion polypeptide comprises in N- to C-terminal direction an antibody light chain variable domain, a multimerization domain and an antibody heavy chain variable domain.

In one embodiment of all aspects the antibody variable domains are fused via peptidic linker to the respective terminus of the multimerization domain.

One aspect as reported herein is a (bivalent) antibody consisting of two fusion polypeptides as reported herein.

In one embodiment (of the (bivalent) antibody)
- one of the CH3 domains has the mutation T366W, and the other CH3 domain has the mutations T366S, L368A, and Y407V (numbering according to the Kabat EU index),
   and
- one of the CH3 domains additionally has the mutation Y349C, and the other CH3 domain additionally has the mutation E356C or S354C (numbering according to the Kabat EU index).

In one embodiment the antibody is a bispecific antibody.

In one embodiment the fusion polypeptide or the antibody specifically binds one antigen selected from the group consisting of VEGF, ANG2, PDGF, and IL-1beta.

In one embodiment the antibody specifically binds two antigens independently of each other selected from the group consisting of VEGF, ANG2, PDGF, and IL-1beta.

In one embodiment the CH3 domains are human CH3 domains. In one preferred embodiment the CH3 domains are human IgG1 CH3 domains. In one embodiment each of the multimerization domains is a pair of a human IgG1 CH2-CH3 domain. In one embodiment the CH1 domain is a human CH1 domain and the CL domain is a human CL domain. In one preferred embodiment the CH1 domain is a human IgG1 CH1 domain and the CL domain is a human kappa or lambda CL domain.

In one embodiment of all aspects as reported herein, each of the multimerization domains is an antibody CH3 domain containing a non-natural cysteine residue. In one embodiment, each of the CH3 domains contains the mutation H433C (numbering according to the Kabat EU Index). In one embodiment, the (N-terminal part of the) heavy chain variable domain of each polypeptide comprises in framework region 3 (FR3) a cysteine residue. In one embodiment, the (N-terminal part of the) heavy chain variable domain of each polypeptide comprises a cysteine residue at position 82b (numbering according to Kabat). In one embodiment, the first part of the first binding site (the heavy chain variable domain of the first binding site) comprises a cysteine residue at position 82b, the first part of the second binding site (the heavy chain variable domain of the second binding site) comprises a cysteine residue at position 82b (numbering according to Kabat), and each of the CH3 domains comprises a cysteine residue at position 433 (numbering according to Kabat EU index).

In one embodiment of all aspects as reported herein, a peptidic linker is present between each part of a binding site and the respective multimerization domain. In one embodiment, each of the polypeptides comprises in N-terminal to C-terminal direction a part of a binding site, a first peptidic linker, a multimerization domain, a second peptidic linker, and a part of a binding site. In one embodiment, the first and the second peptidic linkers have the amino acid sequence SEQ ID NO: 01 and SEQ ID NO: 02. In one embodiment, the first and the second peptidic linkers have the amino acid sequence SEQ ID NO: 03 and SEQ ID NO: 04. In one embodiment, the first and the second peptidic linkers have the amino acid sequence SEQ ID NO: 05 and SEQ ID NO: 06. In one embodiment, the first and the second peptidic linkers have the amino acid sequence SEQ ID NO: 07 and SEQ ID NO: 08.

In one embodiment of all aspects as reported herein, the first binding site and the second binding site each is an antibody binding site (pair of heavy chain variable domain and light chain variable domain).

In one embodiment of all aspects as reported herein, the first part of the first binding site is an antibody light chain variable domain and the second part of the first binding site is an antibody heavy chain variable domain, and the first part of the second binding site is an antibody light chain variable domain and the second part of the second binding site is an antibody heavy chain variable domain.

In one embodiment of all aspects as reported herein, the first part of the first binding site is an antibody light chain variable domain and the second part of the first binding site is an antibody heavy chain variable domain, and the first part of the second binding site is an antibody heavy chain variable domain and the second part of the second binding site is an antibody light chain variable domain.

In one embodiment of all aspects as reported herein, the first part of the first binding site is an antibody heavy chain variable domain and the second part of the first binding site is an antibody light chain variable domain, and the first part of the second binding site is an antibody heavy chain variable domain and the second part of the second binding site is an antibody light chain variable domain.

In one embodiment of all aspects as reported herein, the antibody light and/or heavy chain variable domain is a full-length antibody variable domain. A full-length antibody variable domain comprises the following subdomains (in the N-terminal to C-terminal direction): FR1-CDR1-FR2-CDR2-FR3-CDR3-FR4.

In one embodiment of all aspects as reported herein, the antibody light and/or heavy chain variable domain is a short antibody variable domain. A short antibody variable domain comprises at least the following subdomains (in the N-terminal to C-terminal direction): CDR1-FR2-CDR2-FR3-CDR3. A short antibody variable domain may additionally comprise fragments of the subdomains FR1 (C-terminal fragment) and/or FR4 (N-terminal fragment). In one embodiment, the short antibody variable domain is a full-length antibody variable domain in which two to six C-terminal amino acid residues are absent/deleted (i.e., two to six C-terminal amino acid residues of the FR4 subdomain are not present).

In one embodiment of all aspects as reported herein, the multimerization domain is derived from an antibody CH3 domain. In one embodiment, the multimerization domain is an antibody CH3 domain or a variant thereof or a fragment thereof. In one preferred embodiment, one of the CH3 domains contains the mutation T366W, and the other of the CH3 domains contains the mutations T366S, L368A, and Y407V (numbering according to the Kabat EU Index). In another embodiment, one of the CH3 domains further contains the mutation Y349C, and the other of the CH3 domains further contains the mutation E356C or S354C (numbering according to the Kabat EU Index).

In one embodiment of all aspects as reported herein, the multimerization domains are a domain pair derived from an antibody CH1 domain-CL domain pair. In one preferred embodiment, one of the multimerization domains is an antibody CH1 domain or a variant thereof and the respective other multimerization domain is an antibody CL domain or a variant thereof.

In one embodiment of all aspects as reported herein, the antibody is a bispecific antibody.

In one embodiment of all aspects described herein, the antibody specifically binds one of the antigens selected from the group comprising VEGF, ANG2, PDGF, and IL-1beta.

In one embodiment of all aspects described herein, the antibody specifically binds two antigens selected from the group comprising VEGF, ANG2, PDGF, and IL-1beta.

In one embodiment of all aspects described herein, the antibody specifically binds the antigens VEGF and ANG2, or VEGF and PDGF, or VEGF and IL-1beta.

One aspect as reported herein is a pharmaceutical formulation containing the (bivalent) antibody as reported herein.

One aspect as reported herein is the (bivalent) antibody as reported herein for use in the treatment of an ocular vascular disease.

In one embodiment the ocular vascular disease is age-related macular degeneration or diabetic retinopathy.

### Detailed Description of the Invention

A novel antibody format is reported herein which can be used, among other things, as basic format for producing novel therapeutic agents against age-related macular degeneration, and which has in general improved properties for ophthalmology.

The antibody format reported herein, the Ophthabody, is a novel bivalent, bispecific binding molecule having reduced molecular weight in comparison to a traditional four-chain full-length antibodies and has improved properties compared to other low molecular weight antibody formats.

### I. DEFINITIONS

Herein the amino acid positions of all constant antibody domains (CH1, hinge, CH2, CH3) are denoted according to the numbering system of Kabat (Kabat, et al., Sequences of Proteins of Immunological Interest, 5th Ed., Public Health Service, National Institutes of Health, Bethesda, MD (1991)), and the numbering is referred to as "numbering according to Kabat". In particular, the Kabat numbering system (see pages 647-660 in Kabat, et al., Sequences of Proteins of Immunological Interest, 5th Ed., Public Health Service, National Institutes of Health, Bethesda, MD (1991)) is used for numbering the light constant antibody domain (CL) of the kappa and lambda isotypes, and the Kabat EU numbering index is used for the constant domains of the heavy antibody chain (see pages 661-723; CH1, hinge, CH2, and CH3).

It is herewith expressly stated that the term "comprising" as used herein comprises the term "consisting of'. Thus, all aspects and embodiments that contain the term "comprising" are likewise disclosed with the term "consisting of'.

The term "about" denotes a range of +/- 20 % of the thereafter following numerical value. In one embodiment the term about denotes a range of +/- 10 % of the thereafter following numerical value. In one embodiment the term about denotes a range of +/-5 % of the thereafter following numerical value.

The term "antibody" herein is used in the broadest sense and encompasses various antibody structures, including but not limited to monoclonal antibodies, multispecific antibodies (e.g. bispecific antibodies, trispecific antibodies), and antibody fragments so long as they exhibit the desired antigen- and/or protein A and/or FcRn-binding activity.

The term "antibody binding site" denotes the amino acid residues of an antibody that are responsible for antigen binding. Generally this is a pair of an antibody heavy chain variable domain and antibody light chain variable domain. The antigen-binding site of an antibody comprises amino acid residues from the "hypervariable regions" or "HVRs". "Framework" or "FR" regions are those variable domain regions other than the hypervariable region residues as herein defined. Therefore, the light and heavy chain variable domains of an antibody comprise from N- to C-terminus the regions FR1, CDR1, FR2, CDR2, FR3, CDR3, and FR4 (immunoglobulin framework). Especially, the CDR3 region of the heavy chain is the region, which contributes most to antigen binding and defines the antibody.

The term "binding (to an antigen)" denotes the binding of an antibody to its antigen in an in vitro assay, in one embodiment in a binding assay in which the antibody is bound to a surface and binding of the antigen to the antibody is measured by Surface Plasmon Resonance (SPR). Binding means a binding affinity (K_{D}) of 10⁻⁸ M or less, in some embodiments of 10⁻¹³ to 10⁻⁸ M, in some embodiments of 10⁻¹³ to 10⁻⁹ M.

Binding can be investigated by a BIAcore assay (GE Healthcare Biosensor AB, Uppsala, Sweden). The affinity of the binding is defined by the terms ka (rate constant for the association of the antibody from the antibody/antigen complex), k_{d} (dissociation constant), and K_{D} (k_{d}/kₐ).

The term "binding site" as used herein denotes any proteinaceous entity comprising two separate domains that shows binding specificity to a target. For example an antibody binding site comprises at least three HVRs, for example in case of a naturally occurring, i.e. conventional, antibody by six HVRs.

The term "human IgG1 CH1-domain" denotes the part of an antibody heavy chain polypeptide that extends approximately from EU position 118 to EU position 215 (EU numbering system according to Kabat). In one embodiment the CH1 domain has the amino acid sequence of SEQ ID NO: 25: ASTKGPSVFP LAPSSKSTSG GTAALGCLVK DYFPEPVTVS WNSGALTSGV HTFPAVLQSS GLYSLSSVVT VPSSSLGTQT YICNVNHKPS NTKVDKKV.

The term "human IgG1 CH2-domain" denotes the part of an antibody heavy chain polypeptide that extends approximately from EU position 231 to EU position 340 (EU numbering system according to Kabat). In one embodiment the CH2 domain has the amino acid sequence of SEQ ID NO: 23: APELLGG PSVFLFPPKP KDTLMISRTP EVTCVWDVS HEDPEVKFNW YVDGVEVHNA KTKPREEQ E STYRWSVLT VLHQDWLNGK EYKCKVSNKA LPAPIEKTIS KAK.

The term "human IgG1 CH3-domain" denotes the part of an antibody heavy chain polypeptide that extends approximately from EU position 341 to EU position 445. In one embodiment the CH3 domain has the amino acid sequence of SEQ ID NO: 24: GQPREPQ VYTLPPSRDE LTKNQVSLTC LVKGFYPSDI AVEWESNGQP ENNYKTTPPV LDSDGSFFLY SKLTVDKSRW QQGNVFSCSV MHEALHNHYT QKSLSLSP.

The term "human kappa CL-domain" denotes the part of an antibody light chain polypeptide that extends approximately from position 108 to position 214 (Kabat and Kabat EU index). In one embodiment the CL domain has the amino acid sequence of SEQ ID NO: 21: RTVAAPSVFI FPPSDEQLKS GTASVVCLLN NFYPREAKVQ WKVDNALQSG NSQESVTEQD SKDSTYSLSS TLTLSKADYE KHKVYACEVT HQGLSSPVTK SFNRGEC.

The term "human lambda CL-domain" denotes the part of an antibody heavy chain polypeptide that extends approximately from position 108 to position 215 (according to Kabat). In one embodiment the CL domain has the amino acid sequence of SEQ ID NO: 22: QPKAAPSVTL FPPSSEELQA NKATLVCLIS DFYPGAVTVA WKADSSPVKA GVETTTPSKQ SNNKYAASSY LSLTPEQWKS HRSYSCQVTH EGSTVEKTVA PTECS.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG₁, IgG₂, IgG₃, IgG₄, IgA₁, and IgA₂. The heavy chain constant domains that correspond to the different classes of immunoglobulins are called α, δ, ε, γ, and µ, respectively.

The term "cognate VH/VL-pair" denotes a pair of an antibody heavy chain variable domain and an antibody light chain variable domain, which together form an antibody binding site specifically binding to an antigen. The antibody heavy chain variable domain and the antibody light chain variable domain of a cognate VH/VL-pair are from a single antibody. This single antibody can be, e.g., isolated from a single antibody secreting B-cell, or derived from a single human or humanized antibody or from phage display.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The term "full length antibody" denotes an antibody having a structure substantially similar to a native antibody structure or having heavy chains that contain an Fc-region as defined herein. A full-length antibody may comprise further domains, such as e.g. a scFv or a scFab conjugated to one or more of the chains of the full-length antibody. The term full-length antibody also encompasses these conjugates.

The terms "host cell", "host cell line", and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., the CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "hypervariable region" or "HVR", as used herein, refers to each of the regions of an antibody variable domain which are hypervariable in sequence ("complementarity determining regions" or "CDRs") and form structurally defined loops ("hypervariable loops"), and/or contain the antigen-contacting residues ("antigen contacts"). Generally, antibodies comprise six HVRs; three in the VH (H1, H2, H3), and three in the VL (L1, L2, L3). HVRs as denoted herein include
(a) hypervariable loops occurring at amino acid residues 26-32 (L1), 50-52 (L2), 91-96 (L3), 26-32 (H1), 53-55 (H2), and 96-101 (H3) (Chothia, C. and Lesk, A.M., J. Mol. Biol. 196 (1987) 901-917);
(b) CDRs occurring at amino acid residues 24-34 (L1), 50-56 (L2), 89-97 (L3), 31-35b (H1), 50-65 (H2), and 95-102 (H3) (Kabat, E.A. et al., Sequences of Proteins of Immunological Interest, 5th ed. Public Health Service, National Institutes of Health, Bethesda, MD (1991), NIH Publication 91-3242.);
(c) antigen contacts occurring at amino acid residues 27c-36 (L1), 46-55 (L2), 89-96 (L3), 30-35b (H1), 47-58 (H2), and 93-101 (H3) (MacCallum et al. J. Mol. Biol. 262: 732-745 (1996)); and
(d) combinations of (a), (b), and/or (c), including HVR amino acid residues 46-56 (L2), 47-56 (L2), 48-56 (L2), 49-56 (L2), 26-35 (H1), 26-35b (H1), 49-65 (H2), 93-102 (H3), and 94-102 (H3).

In one embodiment, HVR residues comprise those identified elsewhere in the specification.

Unless otherwise indicated, HVR residues and other residues in the variable domain (e.g., FR residues) are numbered herein according to the Kabat EU index numbering system (Kabat et al., *supra*)*.*

An "individual" or "subject" is a mammal. Mammals include, but are not limited to, domesticated animals (e.g. cows, sheep, cats, dogs, and horses), primates (e.g., humans and non-human primates such as monkeys), rabbits, and rodents (e.g., mice and rats). In certain embodiments, the individual or subject is a human.

An "isolated" antibody is one that has been separated from a component of its natural environment. In some embodiments, an antibody is purified to greater than 95 % or 99 % purity as determined by, for example, electrophoretic (e.g., SDS-PAGE, isoelectric focusing (IEF), capillary electrophoresis) or chromatographic (e.g., size-exclusion chromatography or ion exchange or reverse phase HPLC). For review of methods for assessment of antibody purity, see, e.g., Flatman, S. et al., J. Chrom. B 848 (2007) 79-87.

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

The term "ocular vascular disease" includes, but is not limited to intraocular neovascular syndromes such as diabetic retinopathy, diabetic macular edema, retinopathy of prematurity, neovascular glaucoma, retinal vein occlusions, central retinal vein occlusions, macular degeneration, age-related macular degeneration, retinitis pigmentosa, retinal angiomatous proliferation, macular telangectasia, ischemic retinopathy, iris neovascularization, intraocular neovascularization, corneal neovascularization, retinal neovascularization, choroidal neovascularization, and retinal degeneration (see e.g. Garner, A., Vascular diseases, In: Pathobiology of ocular disease, A dynamic approach, Garner, A., and Klintworth, G.K., (eds.), 2nd edition, Marcel Dekker, New York (1994), pp. 1625-1710).

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

The term "peptidic linker" as used herein denotes a peptide with amino acid sequences, which is in one embodiment of synthetic origin. A "peptidic linker" represents a linear chain of amino acid residues. This linear chain of amino acid residues has a length of 1 to 30 residues.

In one embodiment the peptide linker is rich in glycine, glutamine, and/or serine residues. These residues are arranged e.g. in small repetitive units of up to five amino acids, such as GS, GGS, GGGS, and GGGGS. The small repetitive unit may be repeated for one to five times. At the amino- and/or carboxyl-terminal ends of the multimeric unit up to six additional arbitrary, naturally occurring amino acids may be added.

The peptidic linker is in one embodiment a peptide with an amino acid sequence with a length of up to 30 amino acid residues, in one embodiment with a length of 5 to 20 amino acid residues. In one embodiment the peptidic linker is (GxS)n with G = glycine, S = serine, (x = 3, n = 2, 3, 4 or 5) or (x = 4 and n = 2, 3, or 4), in one embodiment with x = 3, n = 2, in one embodiment with x = 4, n = 2. This peptidic linker may nevertheless comprise additional glycine and/or serine residues at one or both of its termini.

Other synthetic peptidic linkers are composed of a single amino acid, which is repeated between 10 to 20 times and may comprise at the amino- and/or carboxyl-terminal end up to six additional arbitrary, naturally occurring amino acids.

Besides synthetic GS-rich peptidic linkers also naturally occurring peptidic linker such as IgG hinge, liker of human P-glycoprotein, C-terminal linker of human replicatin protein A, linker of the parathyroid hormone-related protein, can be used.

All peptidic linkers can be encoded by a nucleic acid molecule and therefore can be recombinantly expressed. As the linkers are themselves peptides, the polypeptide connected by the linker are connected to the linker via a peptide bond that is formed between two amino acids.

The term "recombinant" or "recombinantly produced" denotes polypeptides that are prepared, expressed, created or isolated by recombinant means. This includes polypeptides isolated from a host cell such as a NS0 or CHO cell or from an animal (e.g. a mouse) that is transgenic or polypeptides expressed using a recombinant expression vector transfected into a host cell.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies or Fc-region fusion polypeptides as reported herein are used to delay development of a disease or to slow the progression of a disease.

The term "valent" as used within the current application denotes the presence of a specified number of binding sites in a (antibody) molecule. As such, the terms "bivalent", "tetravalent", and "hexavalent" denote the presence of two binding site, four binding sites, and six binding sites, respectively, in a (antibody) molecule. The bispecific antibodies as reported herein are in one preferred embodiment "bivalent".

The term "variable region" or "variable domain" refer to the domain of an antibody heavy or light chain that is involved in binding of the antibody to its antigen. The variable domains of the heavy chain and light chain (VH and VL, respectively) of an antibody generally have similar structures, with each domain comprising four framework regions (FRs) and three hypervariable regions (HVRs) (see, e.g., Kindt, T.J. et al. Kuby Immunology, 6th ed., W.H. Freeman and Co., N.Y. (2007), page 91). A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano, S. et al., J. Immunol. 150 (1993) 880-887; Clackson, T. et al., Nature 352 (1991) 624-628).

The term "vector", as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors".

### II. THE VITREOUS HUMOR/BODY

The matrix that fills most space in the eye is denoted as vitreous humor/body.

The human vitreous humor is a clear aqueous solution, which fills the posterior compartment of the eye, located between the lens and the retina. It occupies about 80% of the volume of the eyeball and comprises 99% water but has a gel-like structure at birth due to a network of collagen fibrils and large molecules of hyaluronic acid. Its volume is bout 4-5 ml (Beauthier, J.P., (2008) Quelques aspects biochimiques de l'e 'volution post mortem. In: De Boeck Universite ' [Ed]. Traite ' de me 'de- cine le gale. Bruxelles: 715-725.). Vitreous humor contains several low molecular weight solutes including inorganic salts, sugars and ascorbic acid. The total concentration of protein in human vitreous is approximately 1200 µg/ml, of which collagen accounts for 180 µg/ml (see e.g. Aretz, S., et al., Prot. Sci. 11 (2013) 22; Theocharis, A.D., et al., Biochim. 84 (2002) 1237-1243). An average protein concentration of the healthy vitreous humor of 0.5 mg/mL, consisting largely of albumin (60-70%) is reported by Angi, M., et al. (Hindawi Publishing Corporation, Mediators of Inflammation, Volume 2012, Article ID 148039). Further it is reported therein that components of the vitreous humor are globulins, coagulation proteins, complement factors, and low-molecular-weight proteins (Ulrich, J.N., et al., Clin. Exp. Ophthalmol. 36 (2008) 431-436). The ciliary body provides a constant fluid exchange by diffusion, ultrafiltration, and active transport of aqueous fluid into the posterior segment (Bishop, P.N., Eye, 16 (2002) 454-460). Proteins may accumulate in the vitreous by local secretion (e.g., glycoprotein), filtration from blood (e.g., albumin), or diffusion from the surrounding tissues (Wu, C.W., Am. J. Ophthalmol., 137 (2004) 655-661). Because of the close contact between the vitreous and the inner retina, physiological and pathological conditions of the retina affect both the proteome and the biochemical properties of the vitreous humor.

### III. MACULAR DEGENERATION

Ophthalmology is a branch of medicine concerned with diseases of the eye and the treatment thereof. In the field of ophthalmology, there are numerous serious diseases which greatly influence the eyesight of affected persons, frequently resulting in blindness. Diabetic macular edema (DME), diabetic retinopathy (DR), and age-related macular degeneration (AMD) affect almost 5 million persons in Germany alone, and the number of affected persons is continuously increasing ([1]). AMD is the third most common eye disease worldwide. It results in severe impairment of the vision, and in 8.7 % of cases even leads to total blindness ([2],[3]). In industrialized countries it is the most common eye disease in persons older than 50 years of age, causing irreversible blindness ([4],[5]). It is projected that 196 million persons will be affected by AMD by the year 2020 ([6]).

AMD is a chronically progressive disease, which may be divided into several stages. In the early stage, changes in the retinal pigment epithelium (RPE) due to aging processes, and subretinal deposits of catabolites (glands) between the retinal pigment epithelium and Bruch's membrane, which represents a limiting membrane between the choroidea (choroidal tissue) and the RPE, occur. In the progression, two late stages of AMD may arise. In a certain stage, the disease may result in slow, progressive dry AMD, also referred to as geographic atrophy. This results in degradation of the RPE in the "yellow spot" (Latin: macula lutea), the point of sharpest vision, and breakdown of the blood vessels in the choroidea. A less common stage is rapid, progressive wet AMD, which is characterized by choroidal neovascularization, an ingrowth of new blood vessels in the subretinal space. This results in the escape of plasma and blood into the surrounding tissue ([4],[7]). Both dry AMD and wet AMD result in the death of the photoreceptors, causing loss of central visual acuity.

There are various suspected causes of AMD. In addition to nonspecific, age-related changes of Bruch's membrane, of the retinal pigment epithelium, and of the photoreceptors, primarily oxidative stress, malfunctions of the complement system, localized inflammation, reduction in the lysosomal degradation with accompanying accumulation of retinal toxins, and occurrence of overexpression of the vascular endothelial growth factor (VEGF) have been discussed. An increased VEGF concentration has been detected in the aqueous humor and in the retina of patients with choroidal neovascularization. In addition, numerous in vivo models have shown a relationship between the development of choroidal neovascularization and VEGF expression ([4]).

VEGF represents the primary focus of the current AMD therapies ([7]). VEGF inhibition may halt or at least delay progression of the disease ([8]). This generally requires monthly intravitreal (into the vitreous body) injection of the VEGF inhibitors into the vitreous body. Although available therapies have sometimes resulted in a greatly improved situation for patients, there is still a great need for novel, optimized therapies.

Current clinical studies are focused on molecules which inhibit VEGF and other angiogenic growth factors such as angiopoetin-2 (ANG2). Studies also indicate increased risk of hemorrhaging in the body, a potential side effect of the anti-VEGF therapies ([9]).

### IV. INTRAVITREAL HALF-LIFE

The intravitreal half-life of medicaments, and thereby the number of intravitreal injections, is determined amongst other things by the diffusion properties of the medicaments in the eye and its stability.

The diffusion properties are defined mainly by the molecular weight and the hydrodynamic radius of the molecules. These influence the migration speed of the molecules from the eye into systemic circulation. This is in particular relevant for AMD patients, in which the blood-retina barrier is no longer completely intact. Although the residence time in the eye would be longer if molecules having a higher molecular weight were injected, at the same time the duration of systemic exposure to the medicaments would be increased. This could promote the onset of side effects. The systemic exposure period is a function primarily of the molecular weight of the injected molecules. Molecules having a molecular weight of less than 60 kDa are rapidly excreted via the kidneys; larger molecules spend a longer time in systemic circulation. The most efficient filtration is observed for a molecular weight of < 30 kDa ([10]).

The thermal stability determines how long compounds remain in the correctly folded state under in vivo conditions. Comparing the half-life of two molecules differing only in their thermal stability will show that the molecule with the higher thermal stability maintains its activity for a longer time.

With a long intravitreal half-life less frequent injections are required, with a short half-life in the systemic circulation a low system exposure can be effected, and with the combination of both an increased efficacy and reduced side effects are expected.

A long intravitreal half-life can be achieved by
- high molecular weight (IgGs, addition of e.g. PEG to smaller formats such as diabodies, Fabs etc.),
- high affinity and avidity to target (lower efficient drug concentration results in less frequent dosing),
- high thermal stability at 37°C,
- decreasing diffusion of the molecule across vitreous humor and blood retina barrier (BRB),
- optimal charge or pI.

Rapid systemic clearance can be achieved by
- engineering the Fc-region for reduced FcRn binding,
- low(er) molecular weight (Fab, Diabody, DARPINs),
- low administered doses (dose also depends on affinity).

The intravitreal half-life and durability of a drug can be increased by different means, such as amongst others for example an increase of the hydrodynamic radius of the drug (thereby slowing down the diffusion from the eye), a high affinity of the drug to its target (thereby reducing the dissociation of drug-target complexes), a high (thermal) degradation stability in the eye, and a high injectable dose.

The main factors thought to influencing durability are the dose (an increase of the applicable dose adds positively to durability), the half-life (an increase in half-life adds positively to durability) and the affinity to the target (represented by K_{D}) (an increase in affinity adds positively to durability).

### V. ANTIBODY FORMATS

The monovalent, monospecific fragment antigen binding (Fab) includes the complete light chain as well as the VH and CH1 domains of the heavy chain. The Fab has a molecular weight of approximately 50 kDa.

The monovalent, monospecific single-chain variable fragments (scFvs) is a single polypeptide chain comprising the VL and VH domains and a flexible linker in between. The molecular weight of the scFvs is approximately 28 kDa.

The diabody with a molecular weight of approximately 50 kDa is a bivalent, bispecific molecular format which is derived from scFv.

Fragments having a molecular weight less than a full-length monoclonal antibody (molecular weight of approximately 150 kDa) have better tissue penetration. However, the systemic half-life of these molecules is greatly reduced among other factors due to the lower molecular weight. Both the diabody and the scFvs have a short systemic half-life, since they are below the renal filtration threshold ([11],[12],[13]).

Various molecular properties are important in the field of ophthalmology. On the one hand, a high dosing level is desirable in order to introduce as many molecules into the eye as possible. This is important, since presently only 50 µL volumes can be injected intravitreally. Also rapid elimination from the systemic circulation is desirable. In addition, a high affinity for the antigen and intravitreal half-life are important.

### VI. THE ANTIBODY FORMAT AS DESCRIBED HEREIN

The (bivalent) antibody as reported herein is a novel antibody format called "Ophthabody". It has a molecular weight slightly higher than a Fab fragment, and therefore presumably also a somewhat longer intravitreal half-life. On the other hand it should still be small enough to be rapidly removed from the systemic circulation via the kidneys. The low molecular weight and the molecular structure in combination with a high thermal stability result in improved properties. Without being bound by this theory, the lower molecular weight in comparison to an IgG might also results in improved tissue penetration.

The Ophthabody is made up of two different polypeptide chains. Each of them comprises three domains, which can be conjugated either directly to each other or via peptidic linkers. The N-terminal and C-terminal domains of each polypeptide or the corresponding domains of the polypeptides form a binding site. Thus, the Ophthabody has two binding sites, i.e. it is bivalent. Therefore, the Ophthabody can be monospecific or bispecific. The third and central domain of each of the chains is a multimerization domain. Via this domain the two polypeptides associate with each other. Such a multimerization domain can be, for example, a leucine zipper domain, a helix-loop-helix domain, a PB1 domain, a pair of CH1-CL domains, and a pair of CH3 domains ([14]). In no case the dimerization domain is a pair of antibody hinge region polypeptides.

**Table: Comparison of the properties of different antibody formats (also see [15],[16]).**

| | **IgG** | **Fab** | **Ophthabody** | **scFv** | **diabody** |
|---|---|---|---|---|---|
| **molecular weight [kDa]** | approx. 150 | approx. 50 | 50 < x << 150 | approx. 28 | approx. 50 |
| **intravitreal half-life** | approx. 10 d | approx. 7 d | 7d < x < 10d | << 7 d | approx. 7 d |
| **systemic half-life** | several days | approx. in the range of hours | hours < x < days | in the range of minutes | comparable to Fab |
| **valencies** | two | one | two | one | two |
| **specificities** | mono - bi | mono | mono - bi | mono | mono - bi |
| **thermal stability (Tₘ)** | approx. 58-67°C | approx. 58-67°C | 46-59°C | approx. 45°C (dependent on V_{H}/V_{L}) | approx. 45°C (dependent on V_{HS}/V_{LS}) |

In an example, angiopoietin-2 was selected as antigen 1, and VEGF was selected as antigen 2. Different antibody variants in the herein reported Ophthabody format were designed resulting in 7 different proteins, with protein I representing the base variant.

| variant | first part of binding site | peptidic linker | multimerization domain | peptidic linker | second part of binding site | C-CAP |
|---|---|---|---|---|---|---|
| I HA | VH | GGP | CH3 | GGG | VL | ASEPEA |
| II HA | VH | GGP | CH3 | GGG | VL | GSEPEA |
| III HA | VH | GGGSP | CH3 | GGGGS | VL | ASEPEA |
| IV HA | VH | GGGSP | CH3 | GGGGS | VL | GSEPEA |
| V HA | VH | PGP | CH3 | GPG | VL | GSEPEA |
| VI HA | VH | PPS | CH3 | PPG | VL | GSEPEA |
| VII HA | VH-C variant | G--GP | CH3-C variant | GGG | VL | GSEPEA |

| SEQ ID NO: | first part of binding site | peptidic linker | multimerization domain | peptidic linker | second part of binding site | C-CAP |
|---|---|---|---|---|---|---|
| I HA | 17 | 01 | 10 | 02 | 19 | 20 |
| II HA | 17 | 01 | 10 | 02 | 19 | 09 |
| III HA | 17 | 03 | 10 | 04 | 19 | 20 |
| IV HA | 17 | 03 | 10 | 04 | 19 | 09 |
| V HA | 17 | 05 | 10 | 06 | 19 | 09 |
| VI HA | 17 | 07 | 10 | 08 | 19 | 09 |
| VII HA | 18 | 01 | 12 | 02 | 19 | 09 |

| variant | first part of binding site | peptidic linker | multimerization domain | peptidic linker | second part of binding site | C-CAP |
|---|---|---|---|---|---|---|
| IKV | VH | GGP | CH3 | GGG | VL | AS |
| II KV | VH | GGP | CH3 | GGG | VL | GS |
| III KV | VH | GGGSP | CH3 | GGGGS | VL | AS |
| IV KV | VH | GGGSP | CH3 | GGGGS | VL | GS |
| V KV | VH | PGGP | CH3 | GPG | VL | GS |
| VI KV | VH | PPS | CH3 | PPG | VL | GS |
| VII KV | VH-C variant | GGP | CH3-C variant | GGG | VL | GS |

| SEQ ID NO: | first part of binding site | peptidic linker | multimerization domain | peptidic linker | second part of binding site | C-CAP |
|---|---|---|---|---|---|---|
| I HA | 14 | 01 | 11 | 02 | 16 | AS |
| II HA | 14 | 01 | 11 | 02 | 16 | GS |
| III HA | 14 | 03 | 11 | 04 | 16 | AS |
| IV HA | 14 | 03 | 11 | 04 | 16 | GS |
| V HA | 14 | 05 | 11 | 06 | 16 | GS |
| VI HA | 14 | 07 | 11 | 08 | 16 | GS |
| VII HA | 15 | 01 | 13 | 02 | 16 | GS |

The corresponding pair of HA (first polypeptide, specifically binding to angiopoietin 2) and KV (second polypeptide, specifically binding to VEGF) associate with each other via their CH3 domains and form an Ophthabody. In variant VII additional cysteine residues were introduced to form a stabilizing intra-chain disulfide bridge. The disulfide bridge is formed between the N-terminal domain and the multimerization domain within one polypeptide. Likewise it could also be engineered between the C-terminal domain and the multimerization domain.

Using a luciferase assay (reporter gene assay), testing was conducted to determine how well the Ophthabody variants prevent a signal cascade triggered by VEGF (EC50 = half maximum effective concentration; concentration at which 50 % of the maximum effect is reached).

**Table: The determined relative activity of the protein variants in comparison to bispecific reference IgG in the luciferase assay.**

| **variant** | **EC₅₀ Ophthabody [nM]** | **EC₅₀ reference [nM]** |
|---|---|---|
| I | 1.8 | 2.0 |
| II | 1.8 | 2.0 |
| III | 2.7 | 2.6 |
| IV | 3.0 | 2.6 |
| V | 2.4 | 2.6 |
| VI | 2.2 | 2.6 |
| VII | 2.1 | 2.6 |

Using a kinase receptor activation assay, testing was conducted to determine how well the Ophthabody variants prevent autophosphorylation of the antigen receptor triggered by ANG2 (EC₅₀ = half maximum effective concentration; concentration at which 50 % of the maximum effect is reached).

**Table: The determined relative activity of the protein variants in comparison to bispecific reference IgG in the kinase receptor activation assay.**

| **variant** | **EC₅₀ Ophthabody [nM]** | **EC₅₀ reference [nM]** |
|---|---|---|
| I | 21 | 63 |
| II | 18 | 63 |
| III | 19 | 47 |
| IV | 13 | 47 |
| V | 14 | 47 |
| VI | 18 | 47 |
| VII | 23 | 47 |

All Ophthabody variants can bind both antigens simultaneously.

The thermal stability of the different antibodies has been evaluated by determining the aggregation onset temperature (T_{agg}) and the melting temperature (Tₘ) (see Table below). The respective values for a scFv-CH3-minibody, a diabody and a tandem_di-scFv using the same VH and VL sequences as in the Ophthabody constructs have also been determined.

**Table: The determined melting and aggregation temperatures**

| **Protein** | **Tₘ [°C]** | **T_{agg} [°C]** |
|---|---|---|
| I | 46 | 42 |
| II | 46 | 42 |
| III | 46 | 41 |
| IV | 46 | 41 |
| V | 46 | 39 |
| VI | 46 | 42 |
| VII | 59 | 50 |
| scFv-CH3-minibody | 53 | 50 |
| diabody | 50 | 42 |
| a tandem_di-scFv | 46 | 38 |

It can be seen that the disulfide-stabilized Ophthabody has an increased thermal stability.

All Ophthabody variants were produced with good results

**Table: Summary of production results.**

| | **variant** | | | | | | |
|---|---|---|---|---|---|---|---|
| | **I** | **II** | **III** | **IV** | **V** | **VI** | **VII** |
| **yield [mg] (from 400 mL)** | 2.26 | 4.51 | 3.36 | 4.76 | 3.40 | 5.10 | 3.84 |
| **monomer (SEC)** | 97 | 97 | - | - | 98 | 98 | > 99 |
| **Tₘ [°C]** | 46 | 46 | 46 | 46 | 46 | 46 | 59 |
| **T_{agg} [°C]** | 42 | 42 | 41 | 41 | 39 | 42 | 50 |
| **mass spectrometry** | mass verified | | | | | | |

### Literature Citations

[1] Wolfram, C. and Pfeiffer, N.: Weißbuch zur Situation der ophthalmologischen Versorgung in Deutschland [White Paper on the Situation of Ophthalmological Care in Germany], Deutsche Ophthalmologische Gesellschaft [German Ophthalmological Society], Munich 2012.
[2] World Health Organization: Causes of blindness and visual impairment http://www.who.int/blindness/causes/priority/en/, reviewed on June 10, 2015 at 1:44 p.m.
[3] McHarg, S., et al. (2015). "Age-related macular degeneration and the role of the complement system." Mol Immunol, 67(1): 43-50.
[4] Holz, F. G., et al. (2014). "Recent developments in the treatment of age-related macular degeneration." J Clin Invest, 124(4), 1430-1438.
[5] Fernandez-Robredo, P., et al. (2014). "Current treatment limitations in age-related macular degeneration and future approaches based on cell therapy and tissue engineering." J Ophthalmol, 2014, 510285, 13 pages.
[6] Wong, W. L., et al. (2014). "Global prevalence of age-related macular degeneration and disease burden projection for 2020 and 2040: a systematic review and meta-analysis." The Lancet Global Health, 2(2), e106-e116.
[7] Ishikawa, M., et al. (2015). "Future therapies of wet age-related macular degeneration." J Ophthalmol, 2015, 138070, 10 pages.
[8] Amstutz, C. A., et al. (2015). "Long-Term Outcome in Patients with Intravitreal Anti-VEGF Therapy for Exudative AMD." Klin Monbl Augenheilkd 232(4): 533-537.
[9] Grassmann, F., et al. (2015). "The genetics of age-related macular degeneration (AMD) - Novel targets for designing treatment options?" Eur J Pharm Biopharm, http://dx.doi.org/10.1016/j.ejpb.2015.04.039.
[10] Crommelin, D.J.A., et al.: Pharmaceutical Biotechnology - Fundamentals and Applications, Springer Verlag New York 2013, 4th Edition, pp. 107-109.
[11] Holliger, P. and P. J. Hudson (2005). "Engineered antibody fragments and the rise of single domains." Nat Biotechnol 23(9): 1126-1136.
[12] Wu, A. M. and P. D. Senter (2005). "Arming antibodies: prospects and challenges for immunoconjugates." Nat Biotechnol 23(9): 1137-1146.
[13] Nelson, Aaron L. (2010). "Antibody Fragments: Hope and Hype." mAbs 2(1): 77-83.
[14] McLennan, A., et al.: Molekularbiologie für Biologen, Biochemiker, Pharmazeuten und Mediziner [Molecular Biology for Biologists, Biochemists, Pharmacists, and Physicians], Wiley-VCH Verlag & Co. KGaA Weinheim 2013, 1st Ed., p. 146.
[15] Chames, Patrick et al. (2009) "Therapeutic Antibodies: Successes, Limitations and Hopes for the Future." British Journal of Pharmacology 157(2): 220-233.
[16] Strohl, W.R., et al.: Therapeutic Antibody Engineering - Current and Future Advances Driving the Strongest Growth Area in the Pharmaceutical Industry, Woodhead Publishing 2012, pp. 227-232, 265-286.

### VII. PRODUCTION

The (bivalent) antibody as reported herein is produced by recombinant means. Thus, one aspect as reported herein is a nucleic acid encoding the (bivalent) antibody as reported herein and a further aspect is a cell comprising the nucleic acid encoding a (bivalent) antibody as reported herein. Methods for recombinant production are widely known in the state of the art and comprise protein expression in prokaryotic and eukaryotic cells with subsequent isolation of the (bivalent) antibody and usually purification to a pharmaceutically acceptable purity. For the expression of the (bivalent) antibody as aforementioned in a host cell, nucleic acids encoding the respective (modified) light and heavy chains are inserted into expression vectors by standard methods. Expression is performed in appropriate prokaryotic or eukaryotic host cells like CHO cells, NS0 cells, SP2/0 cells, HEK293 cells, COS cells, PER.C6 cells, yeast, or E.coli cells, and the (bivalent) antibody is recovered from the cells (cultivation supernatant or cells after lysis). General methods for recombinant production of antibodies are well-known in the state of the art and described, for example, in the review articles of Makrides, S.C., Protein Expr. Purif. 17 (1999) 183-202, Geisse, S., et al., Protein Expr. Purif. 8 (1996) 271-282, Kaufman, R.J., Mol. Biotechnol. 16 (2000) 151-160, and Werner, R.G., Drug Res. 48 (1998) 870-880. Antibodies may also be produced using recombinant methods and formulations, e.g., as described in US 4,816,567.

In one embodiment, isolated nucleic acid(s) encoding a (bivalent) antibody as described herein is(are) provided. Such nucleic acid may encode an amino acid sequence comprising the first polypeptide and/or an amino acid sequence comprising the second polypeptide of the (bivalent) antibody. In a further embodiment, one or more vectors (e.g., expression vectors) comprising such nucleic acid are provided. In a further embodiment, a host cell comprising such nucleic acid is provided. In one such embodiment, a host cell comprises (e.g., has been transformed with): (1) a vector comprising a nucleic acid that encodes an amino acid sequence comprising the first polypeptide of the (bivalent) antibody and an amino acid sequence comprising the second polypeptide of the (bivalent) antibody, or (2) a first vector comprising a nucleic acid that encodes an amino acid sequence comprising the first polypeptide of the (bivalent) antibody and a second vector comprising a nucleic acid that encodes an amino acid sequence comprising the second polypeptide of the (bivalent) antibody. In one embodiment, the host cell is eukaryotic, e.g. a Chinese Hamster Ovary (CHO) cell or lymphoid cell (e.g., Y0, NS0, Sp20 cell). In one embodiment, a method of making a (bivalent) antibody as reported herein is provided, wherein the method comprises culturing a host cell comprising a nucleic acid encoding the (bivalent) antibody, as provided above, under conditions suitable for expression of the (bivalent) antibody, and optionally recovering the (bivalent) antibody from the host cell (or host cell culture medium).

Accordingly one aspect as reported herein is a method for the preparation of a (bivalent) antibody as reported herein, comprising the steps of
a) transforming a host cell with one or more vectors comprising nucleic acid molecules encoding the (bivalent) antibody,
b) culturing the host cell under conditions that allow synthesis of the (bivalent) antibody, and
c) recovering the (bivalent) antibody from the culture.

In one embodiment the recovering step under c) includes the use of an Fc-region specific capture reagent. In one embodiment the Fc-region specific capture reagent is used in a bind-and-elute-mode. Examples of such Fc-region specific capture reagents are e.g. Staphylococcus protein A-based affinity chromatography materials.

The (bivalent) antibody is suitably separated from the culture medium by conventional immunoglobulin purification procedures such as, for example, affinity chromatography (protein A-Sepharose), hydroxylapatite chromatography, gel electrophoresis, or dialysis.

DNA and RNA encoding monoclonal antibody binding sites is readily isolated and sequenced using conventional procedures. B-cells or hybridoma cells can serve as a source of such DNA and RNA. Once isolated, the DNA may be inserted into expression vectors, which are then transfected into host cells such as HEK 293 cells, CHO cells, or myeloma cells that do not otherwise produce immunoglobulin protein, to obtain the synthesis of recombinant monoclonal (bivalent) antibodies in the host cells.

Some of the molecules as reported herein provide ease of isolation/purification by comprising Fc-regions that are differentially modified, wherein at least one of the modifications results in i) a differential affinity of the molecule for (Staphylococcal) protein A and ii) a differential affinity of the molecule for the human FcRn, and the molecule is isolable from a disrupted cell, from medium, or from a mixture of molecules based on its affinity for protein A.

Purification of (bivalent) antibody is performed in order to eliminate cellular components or other contaminants, e.g. other cellular nucleic acids or proteins, by standard techniques, including alkaline/SDS treatment, CsCl banding, column chromatography, agarose gel electrophoresis, and others well known in the art (see e.g. Ausubel, F., et al., ed. Current Protocols in Molecular Biology, Greene Publishing and Wiley Interscience, New York (1987)). Different methods are well established and widespread used for protein purification, such as affinity chromatography with microbial proteins (e.g. protein A or protein G affinity chromatography), ion exchange chromatography (e.g. cation exchange (carboxymethyl resins), anion exchange (amino ethyl resins) and mixed-mode exchange), thiophilic adsorption (e.g. with beta-mercaptoethanol and other SH ligands), hydrophobic interaction or aromatic adsorption chromatography (e.g. with phenyl-sepharose, aza-arenophilic resins, or m-aminophenylboronic acid), metal chelate affinity chromatography (e.g. with Ni(II)- and Cu(II)-affinity material), size exclusion chromatography, and electrophoretical methods (such as gel electrophoresis, capillary electrophoresis) (Vijayalakshmi, M.A., Appl. Biochem. Biotech. 75 (1998) 93-102).

Suitable host cells for cloning or expression of bivalent antibody-encoding vectors include prokaryotic or eukaryotic cells described herein. For example, (bivalent) antibody may be produced in bacteria, in particular when glycosylation and Fc effector function are not needed. For expression of polypeptides in bacteria, see, e.g., US 5,648,237, US 5,789,199, and US 5,840,523 (see also Charlton, K.A., In: Methods in Molecular Biology, Vol. 248, Lo, B.K.C. (ed.), Humana Press, Totowa, NJ (2003), pp. 245-254, describing expression of antibody fragments in *E. coli.*)*.* After expression, the (bivalent) antibody may be isolated from the bacterial cell paste in a soluble fraction and can be further purified.

In addition to prokaryotes, eukaryotic microbes such as filamentous fungi or yeast are suitable cloning or expression hosts for (bivalent) antibody-encoding vectors, including fungi and yeast strains whose glycosylation pathways have been "humanized", resulting in the production of a (bivalent) antibody with a partially or fully human glycosylation pattern. See Gerngross, T.U., Nat. Biotech. 22 (2004) 1409-1414; and Li, H. et al., Nat. Biotech. 24 (2006) 210-215.

Suitable host cells for the expression of glycosylated (bivalent) antibody are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts. See, e.g., US 5,959,177, US 6,040,498, US 6,420,548, US 7,125,978, and US 6,417,429 (describing PLANTIBODIES^{™} technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (HEK293 or 293 cells as described, e.g., in Graham, F.L., et al., J. Gen Virol. 36 (1977) 59-74); baby hamster kidney cells (BHK); mouse sertoli cells (TM4 cells as described, e.g., in Mather, J.P., Biol. Reprod. 23 (1980) 243-252); monkey kidney cells (CV1); African green monkey kidney cells (VERO-76); human cervical carcinoma cells (HELA); canine kidney cells (MDCK); buffalo rat liver cells (BRL 3A); human lung cells (W138); human liver cells (Hep G2); mouse mammary tumor (MMT 060562); TRI cells, as described, e.g., in Mather, J.P., et al., Annals N.Y. Acad. Sci. 383 (1982) 44-68; MRC 5 cells; and FS4 cells. Other useful mammalian host cell lines include Chinese hamster ovary (CHO) cells, including DHFR⁻ CHO cells (Urlaub, G., et al., Proc. Natl. Acad. Sci. USA 77 (1980) 4216-4220); and myeloma cell lines such as Y0, NS0 and Sp2/0. For a review of certain mammalian host cell lines suitable for antibody production, see, e.g., Yazaki, P. and Wu, A.M., Methods in Molecular Biology, Vol. 248, Lo, B.K.C. (ed.), Humana Press, Totowa, NJ (2004), pp. 255-268.

### VIII. PHARMACEUTICAL FORMULATION

The (bivalent) antibody as reported herein may have a valuable efficacy/safety profile and may provide benefits for a patient in the need of the respective therapy.

In one aspect, a (bivalent) antibody as reported herein for use as a medicament is provided.

In a further aspect, the invention provides for the use of a (bivalent) antibody in the manufacture or preparation of a medicament. An "individual" according to any embodiments may be a human.

In a further aspect, the invention provides pharmaceutical formulations comprising any of the (bivalent) antibody provided herein, e.g., for use in any of the herein outlined therapeutic methods. In one embodiment, a pharmaceutical formulation comprises any of the (bivalent) antibody provided herein and a pharmaceutically acceptable carrier.

One aspect as reported herein is a pharmaceutical formulation comprising a (bivalent) antibody as reported herein.

Pharmaceutical formulations of a (bivalent) antibody as described herein are prepared by mixing such (bivalent) antibody having the desired degree of purity with one or more optional pharmaceutically acceptable carriers (Remington's Pharmaceutical Sciences, 16th edition, Osol, A. (ed.) (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyl dimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as poly(vinylpyrrolidone); amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e*.*g*. Zn-protein complexes); and/or non-ionic surfactants such as polyethylene glycol (PEG). Exemplary pharmaceutically acceptable carriers herein further include interstitial drug dispersion agents such as soluble neutral-active hyaluronidase glycoproteins (sHASEGP), for example, human soluble PH-20 hyaluronidase glycoproteins, such as rhuPH20 (HYLENEX^{®}, Baxter International, Inc.). Certain exemplary sHASEGPs and methods of use, including rhuPH20, are described in US 2005/0260186 and US 2006/0104968. In one aspect, a sHASEGP is combined with one or more additional glycosaminoglycanases such as chondroitinases.

Exemplary lyophilized antibody formulations are described in US 6,267,958. Aqueous antibody formulations include those described in US 6,171,586 and WO 2006/044908, the latter formulations including a histidine-acetate buffer.

Active ingredients may be entrapped in microcapsules prepared, for example, by coacervation techniques or by interfacial polymerization, for example, hydroxymethylcellulose or gelatin-microcapsules and poly-(methyl methacrylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences, 16th edition, Osol, A. (ed.) (1980).

Sustained-release preparations may be prepared. Suitable examples of sustained-release preparations include semi-permeable matrices of solid hydrophobic polymers containing the (bivalent) antibody, which matrices are in the form of shaped articles, *e.g.* films, or microcapsules.

The formulations to be used for *in vivo* administration are generally sterile. Sterility may be readily accomplished, e.g., by filtration through sterile filtration membranes.

Another aspect as reported herein is the use of a (bivalent) antibody as reported herein for the manufacture of a pharmaceutical formulation. A further aspect as reported herein is a method for the manufacture of a pharmaceutical formulation comprising a (bivalent) antibody as reported herein. In another aspect, a formulation is provided, e.g. a pharmaceutical formulation, containing a (bivalent) antibody as reported herein, formulated together with a pharmaceutical carrier.

A formulation of the present invention can be administered by a variety of methods known in the art. As will be appreciated by the skilled artisan, the route and/or mode of administration will vary depending upon the desired results. To administer a (bivalent) antibody as reported herein by certain routes of administration, it may be necessary to coat the (bivalent) antibody with, or co-administer the (bivalent) antibody with a material to prevent its inactivation. For example, the (bivalent) antibody may be administered to a subject in an appropriate carrier, for example, liposomes, or a diluent. Pharmaceutically acceptable diluents include saline and aqueous buffer solutions. Pharmaceutical carriers include sterile aqueous solutions or dispersions and sterile powders for the extemporaneous preparation of sterile injectable solutions or dispersion. The use of such media and agents for pharmaceutically active substances is known in the art.

Many possible modes of delivery can be used, including, but not limited to intraocular application or topical application. In one embodiment the application is intraocular and includes, but it's not limited to, subconjunctival injection, intracanieral injection, injection into the anterior chamber via the termporai limbus, intrastromal injection, intracorneal injection, subretinal injection, aqueous humor injection, subtenon injection or sustained delivery device, intravitreal injection (e.g., front, mid or back vitreal injection). In one embodiment the application is topical and includes, but it's not limited to eye drops to the cornea.

In one embodiment the (bivalent) antibody or pharmaceutical formulation as reported herein is administered via intravitreal application, e.g. via intravitreal injection. This can be performed in accordance with standard procedures known in the art (see, e.g., Ritter et al., J. Clin. Invest. 116 (2006) 3266-3276, Russelakis-Carneiro et al., Neuropathol. Appl. Neurobiol. 25 (1999) 196-206, and Wray et al., Arch. Neurol. 33 (1976) 183-185).

In some embodiments, therapeutic kits as reported herein can contain one or more doses of a (bivalent) antibody present in a pharmaceutical formulation described herein, a suitable device for intravitreal injection of the pharmaceutical formulation, and an instruction detailing suitable subjects and protocols for carrying out the injection. In these embodiments, the formulations are typically administered to the subject in need of treatment via intravitreal injection. This can be performed in accordance with standard procedures known in the art (see, e.g., Ritter et al., J. Clin. Invest. 116 (2006) 3266-3276, Russelakis-Carneiro et al., Neuropathol. Appl. Neurobiol. 25 (1999) 196-206, and Wray et al., Arch. Neurol. 33 (1976) 183-185).

The formulations may also contain adjuvants such as preservatives, wetting agents, emulsifying agents and dispersing agents. Prevention of presence of microorganisms may be ensured both by sterilization procedures, supra, and by the inclusion of various antibacterial and antifungal agents, for example, paraben, chlorobutanol, phenol, sorbic acid, and the like. It may also be desirable to include isotonic agents, such as sugars, sodium chloride, and the like into the formulations. In addition, prolonged absorption of the injectable pharmaceutical form may be brought about by the inclusion of agents, which delay absorption such as aluminum monostearate and gelatin.

Regardless of the route of administration selected, the (bivalent) antibody as reported herein, which may be used in a suitable hydrated form, and/or the pharmaceutical formulations as reported herein, are formulated into pharmaceutically acceptable dosage forms by conventional methods known to those of skill in the art.

Actual dosage levels of the active ingredients in the pharmaceutical formulations as reported herein may be varied so as to obtain an amount of the active ingredient which is effective to achieve the desired therapeutic response for a particular patient, formulation, and mode of administration, without being toxic to the patient. The selected dosage level will depend upon a variety of pharmacokinetic factors including the activity of the particular formulations employed, the route of administration, the time of administration, the rate of excretion of the particular compound being employed, the duration of the treatment, other drugs, compounds and/or materials used in combination with the particular formulations employed, the age, sex, weight, condition, general health and prior medical history of the patient being treated, and like factors well known in the medical arts.

The formulation must be sterile and fluid to the extent that the formulation is deliverable by syringe. In addition to water, the carrier preferably is an isotonic buffered saline solution.

Proper fluidity can be maintained, for example, by use of coating such as lecithin, by maintenance of required particle size in the case of dispersion and by use of surfactants. In many cases, it is preferable to include isotonic agents, for example, sugars, polyalcohols such as mannitol or sorbitol, and sodium chloride in the formulation.

The formulation can comprise an ophthalmic depot formulation comprising an active agent for subconjunctival administration. The ophthalmic depot formulation comprises microparticles of essentially pure active agent, e.g., the bispecific antibody as reported herein. The microparticles comprising the bispecific antibody as reported herein can be embedded in a biocompatible pharmaceutically acceptable polymer or a lipid-encapsulating agent. The depot formulations may be adapted to release all of substantially all the active material over an extended period of time. The polymer or lipid matrix, if present, may be adapted to degrade sufficiently to be transported from the site of administration after release of all or substantially all of the active agent. The depot formulation can be liquid formulation, comprising a pharmaceutical acceptable polymer and a dissolved or dispersed active agent. Upon injection, the polymer forms a depot at the injections site, e.g. by gelifying or precipitating.

Another aspect as reported herein is the (bivalent) antibody as reported herein for use in the treatment of ocular vascular diseases.

Another aspect as reported herein is the pharmaceutical formulation as reported herein for use in the treatment of ocular vascular diseases.

Another aspect as reported herein is the use of a (bivalent) antibody as reported herein for the manufacture of a medicament for the treatment of ocular vascular disease.

Another aspect as reported herein is method of treatment of patient suffering from ocular vascular diseases by administering a (bivalent) antibody as reported herein to a patient in the need of such treatment.

### IX. THERAPEUTIC METHODS

Any of the (bivalent) antibodies provided herein may be used in therapeutic methods.

In certain embodiments, a (bivalent) antibody for use in a method of treatment is provided. An "individual" according to any of the above embodiments is in one preferred embodiment a human.

In certain embodiments, a (bivalent) antibody for use in a method of treatment is provided. An "individual" according to any of the embodiments is in one preferred embodiment a human.

A (bivalent) antibody as reported herein would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The (bivalent) antibody need not be, but is optionally formulated with one or more agents currently used to prevent or treat the disorder in question. The effective amount of such other agents depends on the amount of (bivalent) antibody present in the formulation, the type of disorder or treatment, and other factors discussed above. These are generally used in the same dosages and with administration routes as described herein, or about from 1 to 99 % of the dosages described herein, or in any dosage and by any route that is empirically/clinically determined to be appropriate.

For the prevention or treatment of disease, the appropriate dosage of a (bivalent) antibody as reported herein (when used alone or in combination with one or more other additional therapeutic agents) will depend on the type of disease to be treated, the type of (bivalent) antibody, the severity and course of the disease, whether the (bivalent) antibody is administered for preventive or therapeutic purposes, previous therapy, the patient's clinical history and response to the (bivalent) antibody, and the discretion of the attending physician. The (bivalent) antibody is suitably administered to the patient at one time or over a series of treatments. Such doses may be administered intermittently, e.g. every week or every three weeks (e.g. such that the patient receives from about two to about twenty, or *e.g.* about six doses of the antibody). An initial higher loading dose, followed by one or more lower doses may be administered. The progress of this therapy is easily monitored by conventional techniques and assays.

### X. ARTICLES OF MANUFACTURE

In another aspect as reported herein, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a formulation, which is by itself or combined with another formulation effective for treating, preventing and/or diagnosing the condition. At least one active agent in the formulation is a (bivalent) antibody as reported herein. The label or package insert indicates that the formulation is used for treating the condition of choice. Moreover, the article of manufacture may comprise (a) a first container with a formulation contained therein, wherein the formulation comprises a (bivalent) antibody as reported herein; and (b) a second container with a formulation contained therein, wherein the formulation comprises a further therapeutic agent. The article of manufacture in this embodiment as reported herein may further comprise a package insert indicating that the formulations can be used to treat a particular condition. Alternatively, or additionally, the article of manufacture may further comprise a second (or third) container comprising a pharmaceutically acceptable buffer. It may further include other materials desirable from a commercial and user standpoint, including other buffers, diluents, filters, needles, and syringes.

### XI. MODIFICATIONS

In a further aspect, a (bivalent) antibody according to any of the above embodiments may incorporate any of the features, singly or in combination, as described in Sections 1-5 below:

### 1. Antibody Affinity

In certain embodiments, the (bivalent) antibody provided herein has an equilibrium dissociation constant (K_{D}) of ≤ 100 nM, ≤ 10 nM (e.g. 10⁻⁷ M or less, e.g. from 10⁻⁷ M to 10⁻¹³ M, e.g., from 10⁻⁸ M to 10⁻¹³ M) for any of its targets.

In one embodiment, K_{D} is measured using a BIACORE^{®} surface plasmon resonance assay. For example, an assay using a BIACORE^{®}-2000 or a BIACORE^{®}-3000 (GE Healthcare Inc., Piscataway, NJ) is performed at 25 °C with immobilized antigen CM5 chips at ~10 response units (RU). In one embodiment, carboxymethylated dextran biosensor chips (CM5, GE Healthcare Inc.) are activated with *N*-ethyl-*N'-*(3-dimethylaminopropyl)-carbodiimide hydrochloride (EDC) and *N-*hydroxysuccinimide (NHS) according to the supplier's instructions. Antigen is diluted with 10 mM sodium acetate, pH 4.8, to 5 µg/mL (~ 0.2 µM) before injection at a flow rate of 5 µL/minute to achieve approximately 10 response units (RU) of coupled protein. Following the injection of antigen, 1 M ethanolamine is injected to block non-reacted groups. For kinetics measurements, two-fold serial dilutions of Fab (0.78 nM to 500 nM) are injected in PBS with 0.05 % polysorbate 20 (TWEEN-20^{™}) surfactant (PBST) at 25 °C at a flow rate of approximately 25 µL/min. Association rates (kₒₙ) and dissociation rates (k_{off}) are calculated using a simple one-to-one Langmuir binding model (BIACORE^{®} Evaluation Software version 3.2) by simultaneously fitting the association and dissociation sensorgrams. The equilibrium dissociation constant (K_{D}) is calculated as the ratio k_{off}/kₒₙ (see, e.g., Chen, Y. et al., J. Mol. Biol. 293 (1999) 865-881). If the on-rate exceeds 10⁶ M⁻¹ s⁻¹ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25 °C of a 20 nM anti-antigen antibody (Fab form) in PBS, pH 7.2, in the presence of increasing concentrations of antigen as measured in a spectrometer, such as a stop-flow equipped spectrophotometer (Aviv Instruments) or a 8000-series SLM-AMINCO^{™} spectrophotometer (ThermoSpectronic) with a stirred cuvette.

### 2. Chimeric and Humanized Binding Sites

In certain embodiments, a (bivalent) antibody provided herein comprises one or two antibody binding sites of a chimeric or humanized antibody.

Certain chimeric antibodies are described, e.g., in US 4,816,567; and Morrison, S.L., et al., Proc. Natl. Acad. Sci. USA 81 (1984) 6851-6855). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro, J.C. and Fransson, J., Front. Biosci. 13 (2008) 1619-1633, and are further described, e.g., in Riechmann, I., et al., Nature 332 (1988) 323-329; Queen, C., et al., Proc. Natl. Acad. Sci. USA 86 (1989) 10029-10033; US 5,821,337, US 7,527,791, US 6,982,321, and US 7,087,409; Kashmiri, S.V., et al., Methods 36 (2005) 25-34 (describing specificity determining region (SDR) grafting); Padlan, E.A., Mol. Immunol. 28 (1991) 489-498 (describing "resurfacing"); Dall'Acqua, W.F. et al., Methods 36 (2005) 43-60 (describing "FR shuffling"); Osbourn, J. et al., Methods 36 (2005) 61-68; and Klimka, A. et al., Br. J. Cancer 83 (2000) 252-260 (describing the "guided selection" approach to FR shuffling).

Human framework regions that may be used for humanization include but are not limited to: framework regions selected using the "best-fit" method (see, e.g., Sims, M.J., et al., J. Immunol. 151 (1993) 2296-2308; framework regions derived from the consensus sequence of human antibodies of a particular subgroup of light or heavy chain variable regions (see, e.g., Carter, P., et al., Proc. Natl. Acad. Sci. USA 89 (1992) 4285-4289; and Presta, L.G., et al., J. Immunol. 151 (1993) 2623-2632); human mature (somatically mutated) framework regions or human germline framework regions (see, e.g., Almagro, J.C. and Fransson, J., Front. Biosci. 13 (2008) 1619-1633); and framework regions derived from screening FR libraries (see, e.g., Baca, M. et al., J. Biol. Chem. 272 (1997) 10678-10684 and Rosok, M.J. et al., J. Biol. Chem. 271 (19969 22611-22618).

### 3. Human Antibody Binding Sites

In certain embodiments, a (bivalent) antibody provided herein comprises one or two antibody binding sites of a human antibody.

Human antibodies can be produced using various techniques known in the art. Human antibodies are described generally in van Dijk, M.A. and van de Winkel, J.G., Curr. Opin. Pharmacol. 5 (2001) 368-374 and Lonberg, N., Curr. Opin. Immunol. 20 (2008) 450-459.

Human antibodies maybe prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, N., Nat. Biotech. 23 (2005) 1117-1125. See also, e.g., US 6,075,181 and US 6,150,584 describing XENOMOUSE^{™} technology; US 5,770,429 describing HUMAB^{®} technology; US 7,041,870 describing K-M MOUSE^{®} technology, and US 2007/0061900, describing VELOCIMOUSE^{®} technology). Human variable regions from intact antibodies generated by such animals may be further modified, e.g., by combining with a different human constant region.

Human antibodies can also be made by hybridoma-based methods. Human myeloma and mouse-human heteromyeloma cell lines for the production of human monoclonal antibodies have been described (see, e.g., Kozbor, D., J. Immunol. 133 (1984) 3001-3005; Brodeur, B.R., et al., Monoclonal Antibody Production Techniques and Applications, Marcel Dekker, Inc., New York (1987), pp. 51-63; and Boerner, P., et al., J. Immunol. 147 (1991) 86-95). Human antibodies generated via human B-cell hybridoma technology are also described in Li, J.et al., Proc. Natl. Acad. Sci. USA 103 (2006) 3557-3562. Additional methods include those described, for example, in US 7,189,826 (describing production of monoclonal human IgM antibodies from hybridoma cell lines) and Ni, J., Xiandai Mianyixue 26 (2006) 265-268 (describing human-human hybridomas). Human hybridoma technology (Trioma technology) is also described in Vollmers, H.P. and Brandlein, S., Histology and Histopathology 20 (2005) 927-937 and Vollmers, H.P. and Brandlein, S., Methods and Findings in Experimental and Clinical Pharmacology 27 (2005) 185-191.

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

### 4. Library-Derived Antibody Binding Sites

A (bivalent) antibody as reported herein may comprise one or two antibody binding sites of an antibody isolated by screening combinatorial libraries for antibodies with the desired activity or activities.

For example, a variety of methods are known in the art for generating phage display libraries and screening such libraries for antibodies possessing the desired binding characteristics. Such methods are reviewed, e.g., in Hoogenboom, H.R. et al., Methods in Molecular Biology 178 (2001) 1-37 and further described, e.g., in the McCafferty, J. et al., Nature348 (1990) 552-554; Clackson, T. et al., Nature 352 (1991) 624-628; Marks, J.D. et al., J. Mol. Biol. 222 (1992) 581-597; Marks, J.D. and Bradbury, A., Methods in Molecular Biology 248 (2003) 161-175; Sidhu, S.S. et al., J. Mol. Biol. 338 (2004) 299-310; Lee, C.V. et al., J. Mol. Biol. 340 (2004) 1073-1093; Fellouse, F.A., Proc. Natl. Acad. Sci. USA 101 (2004) 12467-12472; and Lee, C.V. et al., J. Immunol. Methods 284 (2004) 119-132.

In certain phage display methods, repertoires of VH and VL genes are separately cloned by polymerase chain reaction (PCR) and recombined randomly in phage libraries, which can then be screened for antigen-binding phage as described in Winter, G., et al., Ann. Rev. Immunol. 12 (1994) 433-455.Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self-antigens without any immunization as described by Griffiths, A.D., et al., EMBO J. 12 (1993) 725-734. Finally, naive libraries can also be made synthetically by cloning non-rearranged V-gene segments from stem cells, and using PCR primers containing random sequence to encode the highly variable CDR3 regions and to accomplish rearrangement *in vitro*, as described by Hoogenboom, H.R. and Winter, G., J. Mol. Biol. 227 (1992) 381-388. Patent publications describing human antibody phage libraries include, for example: US 5,750,373, and US 2005/0079574, US 2005/0119455, US 2005/0266000, US 2007/0117126, US 2007/0160598, US 2007/0237764, US 2007/0292936, and US 2009/0002360.

Antibodies or antibody fragments isolated from human antibody libraries are considered human antibodies or human antibody fragments herein.

### 5. Bivalent antibody Variants

In certain embodiments, amino acid sequence variants of the (bivalent) antibody provided herein are contemplated. For example, it may be desirable to improve the binding affinity and/or other biological properties of the (bivalent) antibody. Amino acid sequence variants of a (bivalent) antibody may be prepared by introducing appropriate modifications into the nucleotide sequence encoding the (bivalent) antibody, or by peptide synthesis. Such modifications include, for example, deletions from, and/or insertions into and/or substitutions of residues within the amino acid sequences of the (bivalent) antibody. Any combination of deletion, insertion, and substitution can be made to arrive at the final construct, provided that the final construct possesses the desired characteristics, e.g., antigen-binding.

### a) Substitution, Insertion, and Deletion Variants

In certain embodiments, (bivalent) antibody variants having one or more amino acid substitutions are provided. Sites of interest for substitutional mutagenesis include the HVRs and FRs. Conservative substitutions are shown in the Table below under the heading of "preferred substitutions". More substantial changes are provided in the following Table under the heading of "exemplary substitutions", and as further described below in reference to amino acid side chain classes. Amino acid substitutions may be introduced into a (bivalent) antibody of interest and the products screened for a desired activity, e.g., retained/improved antigen binding, decreased immunogenicity, or improved ADCC or CDC.

**TABLE.**

| **Original Residue** | **Exemplary Substitutions** | **Preferred Substitutions** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

One type of substitutional variant involves substituting one or more hypervariable region residues of a parent (bivalent) antibody (e.g. a humanized or human antibody). Generally, the resulting variant(s) selected for further study will have modifications (e.g., improvements) in certain biological properties (e.g., increased affinity, reduced immunogenicity) relative to the parent antibody (bivalent) antibody and/or will have substantially retained certain biological properties of the parent antibody. An exemplary substitutional variant is an affinity matured (bivalent) antibody, which may be conveniently generated, e.g., using phage display-based affinity maturation techniques such as those described herein. Briefly, one or more HVR residues are mutated and the variant (bivalent) antibody displayed on phage and screened for a particular biological activity (e.g. binding affinity).

Alterations (e.g., substitutions) may be made in HVRs, e.g., to improve (bivalent) antibody affinity. Such alterations may be made in HVR "hotspots," i.e., residues encoded by codons that undergo mutation at high frequency during the somatic maturation process (see, e.g., Chowdhury, P.S., Methods Mol. Biol. 207 (2008) 179-196), and/or residues that contact antigen, with the resulting variant VH or VL being tested for binding affinity. Affinity maturation by constructing and reselecting from secondary libraries has been described, e.g., in Hoogenboom, H.R. et al. in Methods in Molecular Biology 178 (2002) 1-37. In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any (bivalent) antibody variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the (bivalent) antibody to bind antigen. For example, conservative alterations (e.g., conservative substitutions as provided herein) that do not substantially reduce binding affinity may be made in HVRs. Such alterations may, for example, be outside of antigen contacting residues in the HVRs. In certain embodiments of the variant VH and VL sequences provided above, each HVR either is unaltered, or contains no more than one, two or three amino acid substitutions.

A useful method for identification of residues or regions of a (bivalent) antibody that may be targeted for mutagenesis is called "alanine scanning mutagenesis" as described by Cunningham, B.C. and Wells, J.A., Science 244 (1989) 1081-1085. In this method, a residue or group of target residues (e.g., charged residues such as arg, asp, his, lys, and glu) are identified and replaced by a neutral or negatively charged amino acid (e.g., alanine or polyalanine) to determine whether the interaction of the (bivalent) antibody with antigen is affected. Further substitutions may be introduced at the amino acid locations demonstrating functional sensitivity to the initial substitutions. Alternatively, or additionally, a crystal structure of an antigen-(bivalent) antibody complex to identify contact points between the (bivalent) antibody and antigen can be used. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include a (bivalent) antibody with an N-terminal methionyl residue. Other insertional variants of the (bivalent) antibody molecule include the fusion to the N- or C-terminus of the (bivalent) antibody to an enzyme (e.g. for ADEPT) or a polypeptide.

### b) Glycosylation variants

In certain embodiments, a (bivalent) antibody provided herein is altered to increase or decrease the extent to which the (bivalent) antibody is glycosylated. Addition or deletion of glycosylation sites to a (bivalent) antibody may be conveniently accomplished by altering the amino acid sequence such that one or more glycosylation sites is created or removed.

In some embodiments, modifications of the oligosaccharide in an antibody as reported herein may be made in order to create antibody variants with certain improved properties.

Native antibodies produced by mammalian cells typically comprise a branched, biantennary oligosaccharide that is generally attached by an N-linkage to Asn297 of the CH2 domain of the Fc-region. See, e.g., Wright, A. and Morrison, S.L., TIBTECH 15 (1997) 26-32. The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure.

Antibody variants are further provided with bisected oligosaccharides, e.g., in which a biantennary oligosaccharide attached to the (bivalent) antibody is bisected by GlcNAc. Such (bivalent) antibody variants may have reduced fucosylation and/or improved ADCC function.

Examples of antibody variants are described, e.g., in WO 2003/011878; US 6,602,684; US 2005/0123546. Antibody variants with at least one galactose residue in the oligosaccharide attached to the Fc-region are also provided. Such antibody variants may have improved CDC function. Such antibody variants are described, e.g., in WO 1997/30087; WO 1998/58964; WO 1999/22764.

### c) Multimerization Domain Variants

In certain embodiments, one or more further amino acid modifications may be introduced into the multimerization domain(s) of a (bivalent) antibody provided herein, thereby generating a multimerization domain variant. The variant may comprise at least a human CH3 domain sequence (*e.g.*, a human IgG1, IgG2, IgG3 or IgG4 CH3 domain) comprising an amino acid modification (*e*.*g*. a substitution/mutation) at one or more amino acid positions.

In certain embodiments, the invention contemplates a (bivalent) antibody variant that possesses some but not all effector functions, which make it a desirable candidate for applications in which the half-life of the (bivalent) antibody *in vivo* is important yet certain effector functions (such as CDC and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcyR binding (hence likely lacking ADCC activity), but retains FcRn binding ability. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch, J.V. and Kinet, J.P., Annu. Rev. Immunol. 9 (1991) 457-492. Non-limiting examples of *in vitro* assays to assess ADCC activity of a molecule of interest are described in US 5,500,362 (see, e.g. Hellstrom, I. et al., Proc. Natl. Acad. Sci. USA 83 (1986) 7059-7063; and Hellstrom, I. et al., Proc. Natl. Acad. Sci. USA 82 (1985) 1499-1502); US 5,821,337 (see Bruggemann, M. et al., J. Exp. Med. 166 (1987) 1351-1361). Alternatively, non-radioactive assays methods may be employed (see, for example, ACTI^{™} non-radioactive cytotoxicity assay for flow cytometry (CellTechnology, Inc. Mountain View, CA; and CytoTox 96^{®} non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed *in vivo*, *e.g.*, in an animal model such as that disclosed in Clynes, R. et al., Proc. Natl. Acad. Sci. USA 95 (1998) 652-656. C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro, H. et al., J. Immunol. Methods 202 (1996) 163-171; Cragg, M.S. et al., Blood 101 (2003) 1045-1052; and Cragg, M.S. and M.J. Glennie, Blood 103 (2004) 2738-2743). FcRn binding and *in vivo* clearance/half-life determinations can also be performed using methods known in the art (see, e.g., Petkova, S.B. et al., Int. Immunol. 18 (2006) 1759-1769).

Bivalent antibody with reduced effector function include those with substitution of one or more of residues 238, 265, 269, 270, 297, 327 and 329, if present (numbering according to Kabat EU index; see US 6,737,056). Such variants include those with substitutions at two or more of amino acid positions 265, 269, 270, 297 and 327, if present (numbering according to Kabat EU index), including the so-called "DANA" Fc-region mutant with substitution of residues 265 and 297 to alanine, if present (numbering according to Kabat EU index; see US 7,332,581).

Certain antibody variants with improved or diminished binding to FcRs are described (see, e.g., US 6,737,056; WO 2004/056312, and Shields, R.L. et al., J. Biol. Chem. 276 (2001) 6591-6604).

In certain embodiments, a (bivalent) antibody variant comprises a CH3 domain with one or more amino acid substitutions, which improve ADCC, e.g., substitutions at positions 298, 333, and/or 334 of the Fc-region (EU numbering of residues).

In some embodiments, alterations are made that result in altered (*i.e.*, either improved or diminished) C1q binding and/or Complement Dependent Cytotoxicity (CDC), e.g., as described in US 6,194,551, WO 99/51642, and Idusogie, E.E. et al., J. Immunol. 164 (2000) 4178-4184.

Antibodies with increased half-lives and improved binding to the neonatal Fc receptor (FcRn), which is responsible for the transfer of maternal IgGs to the fetus (Guyer, R.L. et al., J. Immunol. 117 (1976) 587-593, and Kim, J.K. et al., J. Immunol. 24 (1994) 2429-2434), are described in US 2005/0014934. Those antibodies comprise an Fc-region with one or more substitutions therein, which improve binding of the Fc-region to FcRn. Such Fc-region variants include those with substitutions at one or more of Fc-region residues: 238, 256, 265, 272, 286, 303, 305, 307, 311, 312, 317, 340, 356, 360, 362, 376, 378, 380, 382, 413, 424 or 434, e.g., substitution of Fc-region residue 434 (US 7,371,826).

See also Duncan, A.R. and Winter, G., Nature 322 (1988) 738-740; US 5,648,260; US 5,624,821; and WO 94/29351 concerning other examples of Fc-region variants.

### d) Heterodimerization

There exist several approaches for CH3-modifications to enforce the heterodimerization, which are well described e.g. in WO 96/27011, WO 98/050431, EP 1870459, WO 2007/110205, WO 2007/147901, WO 2009/089004, WO 2010/129304, WO 2011/90754, WO 2011/143545, WO 2012058768, WO 2013157954, WO 2013096291. Typically in all such approaches the first CH3 domain and the second CH3 domains are both engineered in a complementary manner so that each CH3 domain (or the heavy chain comprising it) cannot longer homodimerize with itself but is forced to heterodimerize with the complementary engineered other CH3 domain (so that the first and second CH3 domain heterodimerize and no homodimers between the two first or the two second CH3 domains are formed). These different approaches for improved heavy chain heterodimerization are contemplated as different alternatives in combination with the heavy-light chain modifications (VH and VL exchange/replacement in one binding arm and the introduction of substitutions of charged amino acids with opposite charges in the CH1/CL interface) in the (bivalent) antibody according to the invention which reduce light chain mispairing an Bence-Jones type side products.

In one embodiment of the invention the CH3 domains of said (bivalent) antibody according to the invention can be altered by the "knob-into-holes" technology which is described in detail with several examples in e.g. WO 96/027011, Ridgway, J.B., et al., Protein Eng. 9 (1996) 617-621; and Merchant, A.M., et al., Nat. Biotechnol. 16 (1998) 677-681; WO 98/050431. In this method the interaction surfaces of the two CH3 domains are altered to increase the heterodimerization of both heavy chains containing these two CH3 domains. Each of the two CH3 domains (of the two heavy chains) can be the "knob", while the other is the "hole". The introduction of a disulfide bridge further stabilizes the heterodimers (Merchant, A.M., et al., Nature Biotech. 16 (1998) 677-681; Atwell, S., et al., J. Mol. Biol. 270 (1997) 26-35) and increases the yield.

Thus in one embodiment of the invention said (bivalent) antibody (comprises a CH3 domain in each polypeptide and) is further characterized in that
the first CH3 domain of the first polypeptide of the (bivalent) antibody and the second CH3 domain of the second polypeptide of the (bivalent) antibody each meet at an interface which comprises an original interface between the CH3 domains.
wherein said interface is altered to promote the formation of the (bivalent) antibody, wherein the alteration is characterized in that:
i) the CH3 domain of one polypeptide is altered, so that within the original interface of the CH3 domain of one polypeptide that meets the original interface of the CH3 domain of the other polypeptide within the (bivalent) antibody, an amino acid residue is replaced with an amino acid residue having a larger side chain volume, thereby generating a protuberance within the interface of the CH3 domain of one polypeptide, which is positionable in a cavity within the interface of the CH3 domain of the other polypeptide
   and
ii) the CH3 domain of the other polypeptide is altered, so that within the original interface of the second CH3 domain that meets the original interface of the first CH3 domain within the (bivalent) antibody an amino acid residue is replaced with an amino acid residue having a smaller side chain volume, thereby generating a cavity within the interface of the second CH3 domain within which a protuberance within the interface of the first CH3 domain is positionable.

Preferably said amino acid residue having a larger side chain volume is selected from the group consisting of arginine (R), phenylalanine (F), tyrosine (Y), tryptophan (W).

Preferably said amino acid residue having a smaller side chain volume is selected from the group consisting of alanine (A), serine (S), threonine (T), valine (V).

In one aspect of the invention both CH3 domains are further altered by the introduction of cysteine (C) as amino acid in the corresponding positions of each CH3 domain such that a disulfide bridge between both CH3 domains can be formed.

In one preferred embodiment, said (bivalent) antibody comprises a amino acid T366W mutation in the first CH3 domain of the "knobs chain" and amino acid T366S, L368A, Y407V mutations in the second CH3 domain of the "hole chain". An additional interchain disulfide bridge between the CH3 domains can also be used (Merchant, A.M., et al., Nature Biotech. 16 (1998) 677-681) e.g. by introducing an amino acid Y349C mutation into the CH3 domain of the "hole chain" and an amino acid E356C mutation or an amino acid S354C mutation into the CH3 domain of the "knobs chain".

In one embodiment, said (bivalent) antibody (which comprises a CH3 domain in each heavy chain) comprises amino acid S354C, T366W mutations in one of the two CH3 domains and amino acid Y349C, T366S, L368A, Y407V mutations in the other of the two CH3 domains (the additional amino acid S354C mutation in one CH3 domain and the additional amino acid Y349C mutation in the other CH3 domain forming an interchain disulfide bridge) (numbering according to Kabat).

Other techniques for CH3-modifications to enforcing the heterodimerization are contemplated as alternatives of the invention and described e.g. in WO 96/27011, WO 98/050431, EP 1870459, WO 2007/110205, WO 2007/147901, WO 2009/089004, WO 2010/129304, WO 2011/90754, WO 2011/143545, WO 2012/058768, WO 2013/157954, WO 2013/096291.

In one embodiment the heterodimerization approach described in EP 1 870 459A1, can be used alternatively. This approach is based on the by the introduction of substitutions/mutations of charged amino acids with the opposite charge at specific amino acid positions of the in the CH3/ CH3 domain interface between both polypeptides. One preferred embodiment for said (bivalent) antibody are amino acid R409D; K370E mutations in the first CH3 domain of the (bivalent) antibody and amino acid D399K; E357K mutations in the second CH3 domain of the (bivalent) antibody (numbering according to Kabat EU index).

In another embodiment said (bivalent) antibody comprises a amino acid T366W mutation in the CH3 domain of the "knobs chain" and amino acid T366S, L368A, Y407V mutations in the CH3 domain of the "hole chain" and additionally amino acid R409D; K370E mutations in the CH3 domain of the "knobs chain" and amino acid D399K; E357K mutations in the CH3 domain of the "hole chain".

In another embodiment said (bivalent) antibody comprises amino acid S354C, T366W mutations in one of the two CH3 domains and amino acid Y349C, T366S, L368A, Y407V mutations in the other of the two CH3 domains or said (bivalent) antibody comprises amino acid Y349C, T366W mutations in one of the two CH3 domains and amino acid S354C, T366S, L368A, Y407V mutations in the other of the two CH3 domains and additionally amino acid R409D; K370E mutations in the CH3 domain of the "knobs chain" and amino acid D399K; E357K mutations in the CH3 domain of the "hole chain".

In one embodiment the heterodimerization approach described in WO 2013/157953 can be used alternatively. In one embodiment a first CH3 domain comprises amino acid T366K mutation and a second CH3 domain polypeptide comprises amino acid L351D mutation. In a further embodiment the first CH3 domain comprises further amino acid L351K mutation. In a further embodiment the second CH3 domain comprises further amino acid mutation selected from Y349E, Y349D and L368E (preferably L368E).

In one embodiment the heterodimerization approach described in WO 2012/058768 can be used alternatively. In one embodiment a first CH3 domain comprises amino acid L351Y, Y407A mutations and a second CH3 domain comprises amino acid T366A, K409F mutations. In a further embodiment the second CH3 domain comprises a further amino acid mutation at position T411, D399, S400, F405, N390, or K392 e.g. selected from a) T411 N, T411 R, T411Q, T411 K, T411D, T411E or T411W, b) D399R, D399W, D399Y or D399K, c S400E, S400D, S400R, or S400K F405I, F405M, F405T, F405S, F405V or F405W N390R, N390K orN390D K392V, K392M, K392R, K392L, K392F or K392E. In a further embodiment a first CH3 domain comprises amino acid L351Y, Y407A mutations and a second CH3 domain comprises amino acid T366V, K409F mutations. In a further embodiment a first CH3 domain comprises amino acid Y407A mutations and a second CH3 domain comprises amino acid T366A, K409F mutations. In a further embodiment the second CH3 domain comprises a further amino acid K392E, T411E, D399R and S400R mutations.

In one embodiment the heterodimerization approach described in WO 2011/143545 can be used alternatively e.g. with the amino acid modification at a position selected from the group consisting of 368 and 409.

In one embodiment the heterodimerization approach described in WO 2011/090762 which also uses the knobs-into-holes technology described above can be used alternatively. In one embodiment a first CH3 domain comprises amino acid T366W mutations and a second CH3 domain comprises amino acid Y407A mutations. In one embodiment a first CH3 domain comprises amino acid T366Y mutations and a second CH3 domain comprises amino acid Y407T mutations.

In one embodiment the multimerization domain is of IgG2 isotype and the heterodimerization approach described in WO 2010/129304 can be used alternatively.

In one embodiment the heterodimerization approach described in WO 2009/089004 can be used alternatively. In one embodiment a first CH3 domain comprises amino acid substitution of K392 or N392 with a negative-charged amino acid (e.g. glutamic acid (E), or aspartic acid (D), preferably K392D or N392D) and a second CH3 domain comprises amino acid substitution of D399, E356, D356, or E357 with a positive-charged amino acid (e.g. Lysine (K) or arginine (R), preferably D399K, E356K, D356K, or E357K and more preferably D399K and E356K. In a further embodiment the first CH3 domain further comprises amino acid substitution of K409 or R409 with a negative-charged amino acid (e.g. glutamic acid (E), or aspartic acid (D), preferably K409D or R409D). In a further embodiment the first CH3 domain further or alternatively comprises amino acid substitution of K439 and/or K370 with a negative-charged amino acid (e.g. glutamic acid (E), or aspartic acid (D)).

In one embodiment the heterodimerization approach described in WO2007/147901 can be used alternatively. In one embodiment a first CH3 domain comprises amino acid K253E, D282K, and K322D mutations and a second CH3 domain comprises amino acid D239K, E240K, and K292D mutations.

In one embodiment the heterodimerization approach described in WO 2007/110205 can be used alternatively.

The following examples, sequences and figures are provided to aid the understanding of the present invention, the true scope of which is set forth in the appended claims.

### Description of the Figures

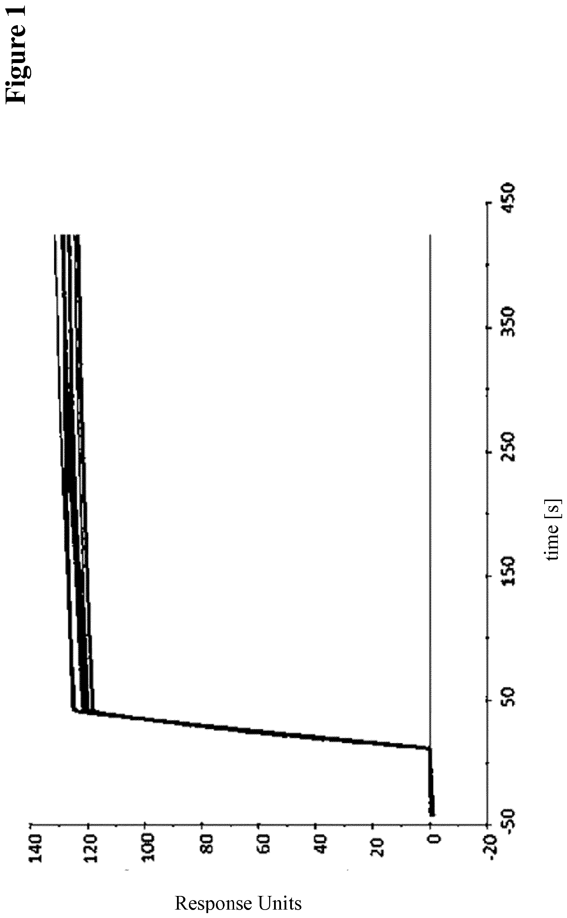

- **Figure 1**: Determination of the binding of the various Ophthabody variants to antigen 2 (VEGF), using BIAcore, over a period of 275 s. The affinity is composed of the "on-rate" and the "off-rate." For the "on-rate," the time for the antibody to bind the antigen is determined. The "off-rate" indicates how long the antibody remains bound until it dissociates from the antigen. In addition, a negative control (baseline) was also measured.

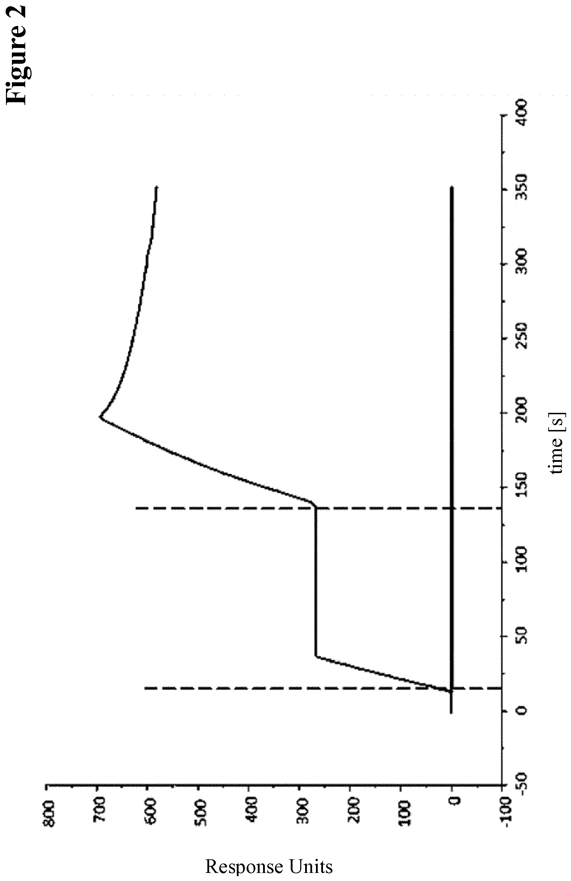

- **Figure 2**: Determination of the binding affinity of protein 1 to antigen 1 and antigen 2, using BIAcore, over a period of 350 s. Antigen 1 was immobilized on the chip, and protein 1 and antigen 2 were injected in succession. The affinity of protein 1 for the antigens is composed of the "on-rate" and the "off-rate." For the "on-rate," the time for the antibody to bind the antigen is determined. The "off-rate" indicates how long the antibody remains bound until it dissociates from the antigen. A negative control (baseline) was measured as reference.

### Examples

### Materials and Methods

### Preparative restriction digest

Initially, 3 µg of the cloning vector was cut with 20 U of the restriction enzymes HindIII-HF and NheI-HF. Likewise, 2 µg of the synthetic gene fragment (Geneart, Regensburg, Germany) to be inserted was cut with 20 U of the restriction enzymes HindIII-HF and NheI-HF. The batches were subsequently incubated at 37°C for 60 minutes. The batches were then analyzed by gel electrophoresis.

### Analytical restriction digest

5 U of the restriction enzymes and an aliquot of the constructs (approximately 500 ng) were used for the batches of the analytical restriction digest. The batches were subsequently incubated at 37°C for 60 minutes. The batches were then analyzed by gel electrophoresis.

### Gel electrophoresis

In each case 1% agarose gel and 0.5 µg of the size standard were used for the analysis of the preparative digest and the analytical restriction digest. The entire digest batch, which had been mixed beforehand with 10× loading buffer, was applied within the scope of the preparative digest. The 1× TBE buffer combined with ethidium bromide was used as the running buffer. The gel electrophoresis was carried out for approximately 45 minutes at a voltage of 100 V. The gel was subsequently analyzed using the UV system and documented.

100 ng of the digest was applied for analysis of the analytical restriction digest. In addition, the same procedure already described for the preparative digest was followed.

### Gel extraction

X-TRACTA Generation II cutters, which remove approximately 120 mg gel, were used for the excision. The High Pure PCR Product Purification Kit from Roche was used according to the manufacturer's specifications to obtain the DNA of the segment of interest from the agarose gel slice. A similar procedure was used to obtain the cut cloning vector.

### Ligation

The Rapid DNA Ligation Kit was used according to the manufacturer's specifications for ligation of the cloning vector and the insert. The cloning vector was dephosphorylated beforehand, using the rAPid Alkaline Phosphatase Kit, to prevent self-ligation. The kit was likewise used according to the manufacturer's specifications. For the re-ligation control, only the cloning vector was added to the ligation batch.

### Transformation

The transformation was carried out according to the manufacturer's specifications to the greatest extent possible. However, the batch composed of cells and the ligation batch was incubated for only 15 minutes on ice. Following thermal shock and the incubation on ice, 300 µL-600 µL S.O.C. medium was added, depending on the size of the transformation batch. The batches were subsequently incubated for 1 h at 37°C and 300 rpm in a Thermomixer. 30 µL-50 µL of the batch was then plated on nutrient agar plates (with 100 µg/mL ampicillin), which were incubated overnight at 37°C.

### Plasmid isolation

The High Pure Plasmid Isolation Kit from Roche was used according to the manufacturer's specifications. Minicultures were inoculated beforehand by picking a colony-forming unit (CFU) from the nutrient agar plate of a construct, and thus inoculating 4 mL of an LB medium combined with ampicillin. This batch was incubated overnight at 37°C and 200 rpm. For producing a maxiculture, 200 µL of the mini culture was used to inoculate 350 mL of LB medium combined with ampicillin. This batch was likewise incubated overnight at 37°C and 200 rpm.

The HiSpeed Plasmid Maxi Kit from Qiagen was used for 150 mL of an overnight culture according to the manufacturer's specifications. For the elution, however, the added TE buffer was diluted 1:4, so that the EDTA was in a low concentration for subsequent experiments. Following the last step of the protocol, the eluate was taken up with the 5 mL syringe for a third time and eluted through the filter into an Eppendorf tube. The tube was centrifuged in a table centrifuge for 2 minutes at 13,000 rpm. The supernatant was transferred to a new Eppendorf tube. The plasmid DNA concentration was subsequently determined using the Nanodrop apparatus.

### DNA precipitation

A specified volume of the recombinant DNA, isolated using the HiSpeed Plasmid Maxi Kit from Qiagen, was precipitated. To this end, 1/10 volume of a 3 M sodium acetate solution, pH 5.2, was added to the isolated DNA and vortexed. 2½ volumes of 100% ethanol were subsequently added and mixed. The batch was incubated for 5 minutes at room temperature. The batch was subsequently centrifuged in the table centrifuge for 5 minutes at 13,000 rpm. The supernatant was discarded. The DNA pellet was combined with 1 mL 70% ethanol, and the batch was shaken back and forth and then incubated for 5 minutes at room temperature. The batch was centrifuged once again in the table centrifuge for 5 minutes at 13,000 rpm. In order to obtain sterile DNA for the transfection, the tubes were transferred to beneath the clean bench, where the supernatant was cautiously removed. The pellet was dried in the open Eppendorf tube. Lastly, the dried pellet was dissolved in double distilled water overnight at 4°C. For determining the DNA concentration, the DNA dissolved in double distilled water was diluted 1:10 in 10 mM Tris-HCl buffer, pH 7.5, and the dilution was measured using the Nanodrop apparatus.

### Cell culture

The procedure was carried out with a HEK293F suspension culture held in F17 medium from Gibco, which had been combined beforehand with 6 mL/L PenStrep. The cells were split every third to fourth day. The cells were agitated at 135 rpm in an incubator at a temperature of 37°C, a CO₂ saturation of 7%, and a relative room humidity of 80%.

### Vitality and cell count determination

The vitality and cell count of the HEK293F suspension culture were determined using the CASY Model TT Cell Counter and Analyzer. The cells were measured in an electrolyte solution (CASY^{®} ton). [32]

### Transient transfection in mammalian cells

The transfection was performed using the 293-Free Transfection Reagent from Novagen according to the manufacturer's specifications. Only one post-transfection step was inserted. 20 mL/L 45% glucose and 20 mL/L L-glutamine were added to the cells on the first day after the transfection. The expressed, secreted proteins were harvested on day 6 or 7. To this end, the entire transfection batch was centrifuged at 4°C and 500 x g for 20 minutes. The supernatant was subsequently transferred into a new vessel and centrifuged for an additional 20 minutes at 4°C and 3452 x g. Lastly, the supernatant together with the expressed, secreted proteins was sterile-filtered.

### CaptureSelect C-tag affinity chromatography

In CaptureSelect C-tag affinity chromatography, a camel antibody fragment immobilized on the column matrix recognizes the C-cap peptide EPEA. The desired protein is thus bound and purified from the supernatant.

### Surface resonance measurement

Surface resonance measurement involves non-cellular interaction assays. Surface resonance measurement allows determination of the affinity with which the antibody binds the antigen. The affinity is composed of the "on-rate" and the "off-rate." For the "on-rate," the time for the antibody to bind the antigen is determined. The "off-rate" indicates how long the antibody remains bound until it dissociates from the antigen. For measuring the simultaneous binding of two antigens, one antigen is immobilized on the chip, and the antibody and the second antigen are injected in succession.

### Cellular assays

In cellular assays, testing is conducted to determine how well the molecule to be analyzed prevents a specific signal cascade in which the molecule to be bound is involved. Within the scope of the cellular assays, HEK cells are used which express a specific receptor as the result of a stable transfection. This receptor binds the antigen which is to be recognized and bound by the antibody to be examined.

### Luciferase assay (reporter gene assay)

In this assay, the antigen 1 to be bound triggers a signal cascade when it binds to the matching receptor. This signal cascade results in the expression of luciferase. The luciferase catalyzes the reaction of 5'-fluoroluciferin to Oxyfluoroluciferin, resulting in luminescence. The relative luminescence units (RLUs) correlate with the luciferase signal. When the antibody binds antigen 1, so that the latter can no longer bind to the receptor, the signal cascade is not triggered and the luminescence signal is absent.

### Kinase receptor activation assay

This assay is based on the autophosphorylation of the antigen receptor during binding of antigen 2. The degree of phosphorylation is determined by ELISA. The multititer plate is covered with "capture antibodies" against the antigen receptor. The cells are incubated with antigen 2 and the antibody, lysed, and placed on the plate. The antigen receptor from the lysate binds to the capture antibodies on the plate. The phosphorylated tyrosins of the receptor are detected by an anti-phosphotyrosine antibody which is coupled to biotin. The biotin group in turn binds to streptavidin, which is coupled to horseradish peroxidase. Lastly, the horseradish peroxidase substrate TMB is added and converted by the peroxidase, resulting in an absorbing substance. The reaction is terminated, and the optical density is measured.

### Recombinant DNA techniques

Standard methods were used to manipulate DNA as described in Sambrook, J. et al., Molecular Cloning: A laboratory manual; Cold Spring Harbor Laboratory Press, Cold Spring Harbor, New York (1989). The molecular biological reagents were used according to the manufacturer's instructions.

### Gene synthesis

Desired gene segments were ordered according to given specifications at Geneart (Regensburg, Germany).

### DNA sequence determination

DNA sequences were determined by double strand sequencing performed at MediGenomix GmbH (Martinsried, Germany) or SequiServe GmbH (Vaterstetten, Germany).

### DNA and protein sequence analysis and sequence data management

The GCG's (Genetics Computer Group, Madison, Wisconsin) software package version 10.2 and Infomax's Vector NT1 Advance suite version 8.0 was used for sequence creation, mapping, analysis, annotation and illustration.

### Expression vectors

For the expression of the described antibodies expression plasmids for transient expression (e.g. in HEK293-F cells) based either on a cDNA organization with or without a CMV-Intron A promoter or on a genomic organization with a CMV promoter were used.

The transcription unit of the antibody gene was composed of the following elements:
- unique restriction site(s) at the 5' end,
- the immediate early enhancer and promoter from the human cytomegalovirus,
- in the case of the cDNA organization the Intron A sequence,
- a 5'-untranslated region of a human immunoglobulin gene,
- a nucleic acid encoding an immunoglobulin heavy chain signal sequence,
- a nucleic acid encoding the human antibody chain (wild-type or with domain exchange) either as cDNA or in genomic organization with the immunoglobulin exon-intron organization,
- a 3' non-translated region with a polyadenylation signal sequence, and
- unique restriction site(s) at the 3' end.

Beside the antibody expression cassette the plasmids contained:
- an origin of replication which allows replication of this plasmid in *E*. *coli*,
- a β-lactamase gene which confers ampicillin resistance in *E. coli.,* and
- the dihydrofolate reductase gene from *Mus musculus* as a selectable marker in eukaryotic cells.

The nucleic acids encoding the antibody chains were generated by PCR and/or gene synthesis and assembled by known recombinant methods and techniques by connection of the according nucleic acid segments e.g. using unique restriction sites in the respective vectors. The subcloned nucleic acid sequences were verified by DNA sequencing. For transient transfections larger quantities of the plasmids were prepared by plasmid preparation from transformed *E. coli* cultures (Nucleobond AX, Macherey-Nagel).

### Cell culture techniques

Standard cell culture techniques were used as described in Current Protocols in Cell Biology (2000), Bonifacino, J.S., Dasso, M., Harford, J.B., Lippincott-Schwartz, J. and Yamada, K.M. (eds.), John Wiley & Sons, Inc.

The bispecific antibodies were expressed by transient co-transfection of the respective expression plasmids in in HEK293-F cells growing in suspension as described below.

### Example 1

### Transfection and protein purification

The co-transfection of two DNA constructs in HEK293-F cells was carried out in each case using the purified, sterile recombinant DNA. The proteins were subsequently purified from the supernatants by affinity chromatography and SEC.

400 mL supernatant of the particular co-transfection was purified in each case. Initially, the protein was purified via CaptureSelect C-tag affinity chromatography (see the following table). The proteins of the eluate were subsequently separated or purified according to size by SEC, and the desired molecular format was isolated.

**Table: Protein yield of the proteins expressed in HEK293F cells after protein purification by CaptureSelect C-tag affinity chromatography and by preparative and analytical SEC**

| **variant** | **Mass [mg]** |
|---|---|
| I | 2.26 |
| II | 4.51 |
| III | 3.36 |
| IV | 4.76 |
| V | 3.40 |
| VI | 5.10 |
| VII | 3.84 |

### Example 2

### Mass spectrometric analysis

The molecular weight of the proteins was analyzed using an electrospray ionization (ESI) mass spectrometer and compared to the theoretical molecular weight determined based on the amino acid sequence. The mass and identity were confirmed for all expressed protein variants.

### Example 3

### Thermal stability determination

The proteins were examined for thermal stability using the Avacta Optim apparatus, and the melting temperature and aggregate temperature were determined.

**Table: Determination of the melting temperature and aggregate temperature of the purified proteins**

| **Protein** | **Tₘ [°C]** | **T_{agg} [°C]** |
|---|---|---|
| I | 46 | 42 |
| II | 46 | 42 |
| III | 46 | 41 |
| IV | 46 | 41 |
| V | 46 | 39 |
| VI | 46 | 42 |
| VII | 59 | 50 |

### Example 4

### Binding assay

The binding assay was performed on a BIAcore apparatus, using the surface resonance method. The BIAcore Assay was used to determine the affinity with which the Ophthabody variants bind antigens 1 and 2. The affinity is composed of the "on-rate" and the "off-rate." The bispecific IgG was once again used as reference standard.

Determination of the binding affinity of the various Ophthabody variants to antigen 1 was carried out over a period of 425 s. Determination of the binding of the various Ophthabody variants to antigen 2 was carried out over a period of 275 s, using the BIAcore apparatus.

### Example 5

### Luciferase assay (reporter gene assay)

**Table: The determined relative activity of the protein variants in comparison to the bispecific reference IgG in the luciferase assay**

| **variant** | **ECso Ophthabody [nM]** | **EC₅₀ reference [nM]** |
|---|---|---|
| I | 1.8 | 2.0 |
| II | 1.8 | 2.0 |
| III | 2.7 | 2.6 |
| IV | 3.0 | 2.6 |
| V | 2.4 | 2.6 |
| VI | 2.2 | 2.6 |
| VII | 2.1 | 2.6 |

### Example 6

### Inhibition of huANG-2 binding to Tie-2 (ELISA)

The interaction ELISA was performed on 384 well microtiter plates (MicroCoat, DE, Cat. No. 464718) at RT. After each incubation step plates were washed 3 times with PBST. ELISA plates were coated with 5 µg/ml Tie-2 protein for 1 hour (h). Thereafter the wells were blocked with PBS supplemented with 0.2% Tween-20 and 2% BSA (Roche Diagnostics GmbH, DE) for 1 h. Dilutions of purified antibodies in PBS were incubated together with 0.2 µg/ml huAngiopoietin-2 (R&D Systems, UK, Cat. No. 623-AN) for 1 h. at RT. After washing a mixture of 0.5 µg/ml biotinylated anti-Angiopoietin-2 clone BAM0981 (R&D Systems, UK) and 1:3000 diluted streptavidin HRP (Roche Diagnostics GmbH, DE, Cat.No. 11089153001) was added for 1 h. Thereafter the plates were washed 3 times with PBST. Plates are developed with freshly prepared ABTS reagent (Roche Diagnostics GmbH, DE, buffer #204 530 001, tablets #11 112 422 001) for 30 minutes at RT. Absorbance was measured at 405 nm and the IC50 was determined.

**Table: The determined relative activity of the protein variants in comparison to the bispecific reference IgG in the kinase receptor activation assay**

| **variant** | **EC₅₀ Ophthabody [nM]** | **EC₅₀ reference [nM]** |
|---|---|---|
| I | 21 | 63 |
| II | 18 | 63 |
| III | 19 | 47 |
| IV | 13 | 47 |
| V | 14 | 47 |
| VI | 18 | 47 |
| VII | 23 | 47 |

### Example 7

### Kinetic characterization

### ANG2:

Binding of the bispecific antibody to human ANG2-RBD-mouse Fc-region fusion was investigated by surface plasmon resonance using a BIACORE T200 instrument (GE Healthcare). Around 4000 RU of anti-mouse Fc-region antibody (10 µg/ml anti-mouse (Fc) antibody) were coupled on a Series S CM5 chip (GE Healthcare BR-1005-30) at pH 5.0 by using an amine coupling kit supplied by the GE Healthcare. HBS-N (10 mM HEPES, 150 mM NaCl pH 7.4, GE Healthcare) was used as running buffer during the immobilization procedure. For the following kinetic characterization, sample and running buffer was HBS-P (10 mM HEPES, 150 mM NaCl pH 7.4, 0.05 % Surfactant P20; GE Healthcare). The flow cell was set to 25 °C - and the sample block set to 12 °C - and primed with running buffer twice prior to kinetic characterization.

Human ANG2-RBD-murine Fc-region fusion was captured by injecting a 1 µg/ml solution for 30 sec. at a flow rate of 5 µl/min. Association was measured by injection of the bispecific antibody in various concentrations in solution for 90 sec. at a flow rate of 90 µl/min starting with 300 nM in serial 1:3 dilutions. The dissociation phase was monitored for up to 600 sec. and triggered by switching from the sample solution to running buffer. All surfaces were regenerated by 60 sec. washing with a 3 M MgCl2 solution at a flow rate of 5 µl/min. Bulk refractive index differences were corrected by subtracting the response obtained from an anti-mouse IgG antibody (Fc) surface. Blank injections were also subtracted (= double referencing). For calculation of KD and other kinetic parameters the Langmuir 1:1 model was VEGF:

Binding of the bispecific antibody to human VEGF isoform 121 was investigated by surface plasmon resonance using a BIACORE T200 instrument (GE Healthcare). An anti-hexa-histidine antibody was coupled on a CM5 chip (GE Healthcare BR-1005-30) according to the manufacturer's instructions by using an amine coupling kit supplied by the GE Healthcare. HBS-N (10 mM HEPES, 150 mM NaCl pH 7.4, GE Healthcare) was used as running buffer during the immobilization procedure. For the following kinetic characterization, sample and running buffer was HBS-P (10 mM HEPES, 150 mM NaCl pH 7.4, 0.05 % Surfactant P20; GE Healthcare). The flow cell was set to 25 °C - and the sample block set to 12 °C - and primed with running buffer twice prior to kinetic characterization.

Histidine-tag comprising human VEGF isoform 121 was captured by injecting a solution for 30 sec. at a flow rate of 5 µl/min. Association was measured by injection of the bispecific antibody in various concentrations in solution for 90 sec. at a flow rate of 90 µl/min starting with 300 nM in serial 1:3 dilutions. The dissociation phase was monitored for up to 600 sec. and triggered by switching from the sample solution to running buffer. All surfaces were regenerated by 60 sec. washing with a 3 M MgCl2 solution at a flow rate of 5 µl/min. Bulk refractive index differences were corrected by subtracting the response obtained from an anti-hexa-histidine antibody surface. Blank injections were also subtracted (= double referencing). For calculation of KD and other kinetic parameters the Langmuir 1:1 model was used.

### Example 8

### Aggregation temperature by Dynamic Light Scattering (DLS)

The temperature at which thermally induced protein aggregation occurs was determined by dynamic light scattering (DLS). DLS yields information on the size distribution of macromolecules in solution, derived from fluctuations of scattered light intensities on a microsecond scale. When samples are heated up gradually, aggregation starts at a certain temperature, giving rise to growing particle sizes. The temperature at which particle sizes begin to increase is defined as the aggregation temperature. Aggregation and denaturation temperatures need not necessarily be identical since denaturation may not necessarily be a prerequisite for aggregation.

For aggregation temperature measurements, a DynaPro DLS plate reader (Wyatt technologies) was used. Preceding the measurement, samples were filtered via 384-well filter plates (Millipore MultiScreen 384-wellFiltration System, 0.45 µm) into optical 384 well plates (Corning #3540). A sample volume of 35 µL was used at a protein concentration of approx. 1 mg/mL in formulation buffer (20 mM citrate, 180 mM sucrose, 20 mM arginine, 0.02 % polysorbate 20). Each well was covered with 20 µL paraffin oil (Sigma) to avoid evaporation. Samples were heated from 25 °C to 80 °C at a rate of 0.05 °C/min and DLS data were acquired continuously for a maximum number of 15 samples per run.

| **variant** | **Mass [mg]** |
|---|---|
| I | 2.26 |
| II | 4.51 |
| III | 3.36 |
| IV | 4.76 |
| V | 3.40 |
| VI | 5.10 |
| VII | 3.84 |

### Example 2

### Mass spectrometric analysis

The molecular weight of the proteins was analyzed using an electrospray ionization (ESI) mass spectrometer and compared to the theoretical molecular weight determined based on the amino acid sequence. The mass and identity were confirmed for all expressed protein variants.

### Example 3

### Thermal stability determination

The proteins were examined for thermal stability using the Avacta Optim apparatus, and the melting temperature and aggregate temperature were determined.

**Table: Determination of the melting temperature and aggregate temperature of the purified proteins**

| **Protein** | **Tₘ [°C]** | **T_{agg} [°C]** |
|---|---|---|
| I | 46 | 42 |
| II | 46 | 42 |
| III | 46 | 41 |
| IV | 46 | 41 |
| V | 46 | 39 |
| VI | 46 | 42 |
| VII | 59 | 50 |

### Example 4

### Binding assay

The binding assay was performed on a BIAcore apparatus, using the surface resonance method. The BIAcore Assay was used to determine the affinity with which the Ophthabody variants bind antigens 1 and 2. The affinity is composed of the "on-rate" and the "off-rate." The bispecific IgG was once again used as reference standard.

Determination of the binding affinity of the various Ophthabody variants to antigen 1 was carried out over a period of 425 s. Determination of the binding of the various Ophthabody variants to antigen 2 was carried out over a period of 275 s, using the BIAcore apparatus.

### Example 5

### Luciferase assay (reporter gene assay)

**Table: The determined relative activity of the protein variants in comparison to the bispecific reference IgG in the luciferase assay**

| **variant** | **EC₅₀ Ophthabody [nM]** | **EC₅₀ reference [nM]** |
|---|---|---|
| I | 1.8 | 2.0 |
| II | 1.8 | 2.0 |
| III | 2.7 | 2.6 |
| IV | 3.0 | 2.6 |
| V | 2.4 | 2.6 |
| VI | 2.2 | 2.6 |
| VII | 2.1 | 2.6 |

### Example 6

### Inhibition of huANG-2 binding to Tie-2 (ELISA)

The interaction ELISA was performed on 384 well microtiter plates (MicroCoat, DE, Cat. No. 464718) at RT. After each incubation step plates were washed 3 times with PBST. ELISA plates were coated with 5 µg/ml Tie-2 protein for 1 hour (h). Thereafter the wells were blocked with PBS supplemented with 0.2% Tween-20 and 2% BSA (Roche Diagnostics GmbH, DE) for 1 h. Dilutions of purified antibodies in PBS were incubated together with 0.2 µg/ml huAngiopoietin-2 (R&D Systems, UK, Cat. No. 623-AN) for 1 h. at RT. After washing a mixture of 0.5 µg/ml biotinylated anti-Angiopoietin-2 clone BAM0981 (R&D Systems, UK) and 1:3000 diluted streptavidin HRP (Roche Diagnostics GmbH, DE, Cat.No.11089153001) was added for 1 h. Thereafter the plates were washed 3 times with PBST. Plates are developed with freshly prepared ABTS reagent (Roche Diagnostics GmbH, DE, buffer #204 530 001, tablets #11 112 422 001) for 30 minutes at RT. Absorbance was measured at 405 nm and the IC50 was determined.

**Table: The determined relative activity of the protein variants in comparison to the bispecific reference IgG in the kinase receptor activation assay**

| **variant** | **EC₅₀ Ophthabody [nM]** | **EC₅₀ reference [nM]** |
|---|---|---|
| I | 21 | 63 |
| II | 18 | 63 |
| III | 19 | 47 |
| IV | 13 | 47 |
| V | 14 | 47 |
| VI | 18 | 47 |
| VII | 23 | 47 |

### Example 7

### Kinetic characterization

### ANG2:

Binding of the bispecific antibody to human ANG2-RBD-mouse Fc-region fusion was investigated by surface plasmon resonance using a BIACORE T200 instrument (GE Healthcare). Around 4000 RU of anti-mouse Fc-region antibody (10 µg/ml anti-mouse (Fc) antibody) were coupled on a Series S CM5 chip (GE Healthcare BR-1005-30) at pH 5.0 by using an amine coupling kit supplied by the GE Healthcare. HBS-N (10 mM HEPES, 150 mM NaCl pH 7.4, GE Healthcare) was used as running buffer during the immobilization procedure. For the following kinetic characterization, sample and running buffer was HBS-P (10 mM HEPES, 150 mM NaCl pH 7.4, 0.05 % Surfactant P20; GE Healthcare). The flow cell was set to 25 °C - and the sample block set to 12 °C - and primed with running buffer twice prior to kinetic characterization.

Human ANG2-RBD-murine Fc-region fusion was captured by injecting a 1 µg/ml solution for 30 sec. at a flow rate of 5 µl/min. Association was measured by injection of the bispecific antibody in various concentrations in solution for 90 sec. at a flow rate of 90 µl/min starting with 300 nM in serial 1:3 dilutions. The dissociation phase was monitored for up to 600 sec. and triggered by switching from the sample solution to running buffer. All surfaces were regenerated by 60 sec. washing with a 3 M MgC12 solution at a flow rate of 5 µl/min. Bulk refractive index differences were corrected by subtracting the response obtained from an anti-mouse IgG antibody (Fc) surface. Blank injections were also subtracted (= double referencing). For calculation of KD and other kinetic parameters the Langmuir 1:1 model was

### VEGF:

Binding of the bispecific antibody to human VEGF isoform 121 was investigated by surface plasmon resonance using a BIACORE T200 instrument (GE Healthcare). An anti-hexa-histidine antibody was coupled on a CM5 chip (GE Healthcare BR-1005-30) according to the manufacturer's instructions by using an amine coupling kit supplied by the GE Healthcare. HBS-N (10 mM HEPES, 150 mM NaCl pH 7.4, GE Healthcare) was used as running buffer during the immobilization procedure. For the following kinetic characterization, sample and running buffer was HBS-P (10 mM HEPES, 150 mM NaCl pH 7.4, 0.05 % Surfactant P20; GE Healthcare). The flow cell was set to 25 °C - and the sample block set to 12 °C - and primed with running buffer twice prior to kinetic characterization.

Histidine-tag comprising human VEGF isoform 121 was captured by injecting a solution for 30 sec. at a flow rate of 5 µl/min. Association was measured by injection of the bispecific antibody in various concentrations in solution for 90 sec. at a flow rate of 90 µl/min starting with 300 nM in serial 1:3 dilutions. The dissociation phase was monitored for up to 600 sec. and triggered by switching from the sample solution to running buffer. All surfaces were regenerated by 60 sec. washing with a 3 M MgCl₂ solution at a flow rate of 5 µl/min. Bulk refractive index differences were corrected by subtracting the response obtained from an anti-hexa-histidine antibody surface. Blank injections were also subtracted (= double referencing). For calculation of KD and other kinetic parameters the Langmuir 1:1 model was used.

### Example 8

### Aggregation temperature by Dynamic Light Scattering (DLS)

The temperature at which thermally induced protein aggregation occurs was determined by dynamic light scattering (DLS). DLS yields information on the size distribution of macromolecules in solution, derived from fluctuations of scattered light intensities on a microsecond scale. When samples are heated up gradually, aggregation starts at a certain temperature, giving rise to growing particle sizes. The temperature at which particle sizes begin to increase is defined as the aggregation temperature. Aggregation and denaturation temperatures need not necessarily be identical since denaturation may not necessarily be a prerequisite for aggregation.

For aggregation temperature measurements, a DynaPro DLS plate reader (Wyatt technologies) was used. Preceding the measurement, samples were filtered via 384-well filter plates (Millipore MultiScreen 384-well Filtration System, 0.45 µm) into optical 384 well plates (Corning #3540). A sample volume of 35 µL was used at a protein concentration of approx. 1 mg/mL in formulation buffer (20 mM citrate, 180 mM sucrose, 20 mM arginine, 0.02 % polysorbate 20). Each well was covered with 20 µL paraffin oil (Sigma) to avoid evaporation. Samples were heated from 25 °C to 80 °C at a rate of 0.05 °C/min and DLS data were acquired continuously for a maximum number of 15 samples per run.

## Claims

1. A fusion polypeptide comprising an antibody heavy chain variable domain, an antibody CH3 domain as multimerization domain and an antibody light chain variable domain,
wherein
- the antibody heavy chain variable domain is fused via a first (peptidic) linker to one terminus of the antibody CH3 multimerization domain,
- the antibody light chain variable domain is fused via a second (peptidic) linker to the respective other terminus of the antibody CH3 multimerization domain, and
- the antibody heavy chain variable domain has an intra-peptidic disulphide bond to the antibody CH3 multimerization domain,
wherein the heavy chain variable domain comprises at position 82b , according to Kabat, a cysteine amino acid residue,
wherein the antibody CH3 domain has at position 433, numbering according to Kabat EU index, a cysteine amino acid residue, wherein the disulfide bond is between the cysteine residue at position 82b of the heavy chain variable domain and the cysteine residue at position 433 of the CH3 domain, and
wherein the N-terminal and C-terminal domains of the fusion polypeptide form a binding site.

2. The fusion polypeptide according to claim 1, wherein the fusion polypeptide comprises in N- to C-terminal direction an antibody heavy chain variable domain, an antibody CH3 multimerization domain and an antibody light chain variable domain.

3. The fusion polypeptide according to claim 1, wherein the fusion polypeptide comprises in N- to C-terminal direction an antibody light chain variable domain, an antibody CH3 multimerization domain and an antibody heavy chain variable domain.

4. The fusion polypeptide according to any one of claims 1 to 3, wherein the first and the second peptidic linkers have the amino acid sequence SEQ ID NO: 01 and SEQ ID NO: 02.

5. The fusion polypeptide according to any one of claims 1 to 3, wherein the first and the second peptidic linkers have the amino acid sequence SEQ ID NO: 03 and SEQ ID NO: 04.

6. The fusion polypeptide according to any one of claims 1 to 3, wherein the first and the second peptidic linkers have the amino acid sequence SEQ ID NO: 05 and SEQ ID NO: 06.

7. The fusion polypeptide according to any one of claims 1 to 3, wherein the first and the second peptidic linkers have the amino acid sequence SEQ ID NO: 07 and SEQ ID NO: 08.

8. An antibody consisting of two fusion polypeptides according to any one of claims 1 to 7.

9. The antibody according to claim 8, wherein
- one of the CH3 domains contains the mutation T366W, and the other of the CH3 domains contains the mutations T366S, L368A, and Y407V (numbering according to the Kabat EU index),
and
- wherein one of the CH3 domains additionally contains the mutation Y349C, and the other of the CH3 domains additionally contains the mutation E356C or S354C (numbering according to the Kabat EU index).

10. The antibody according to any one of claims 8 to 9, wherein the antibody is a bispecific antibody.

11. The antibody according to any one of claims 8 to 10 for use as a medicament.

12. The antibody according to any one of claims 8 to 10 for use in the treatment of an ocular vascular disease.

13. The antibody for use according to claim 12, wherein the ocular vascular disease is age-related macular degeneration or diabetic retinopathy.

14. A pharmaceutical formulation containing the fusion polypeptide according to any one of claims 1 to 7 or the antibody according to any one of claims 8 to 10.

## Patentansprüche

1. Fusionspolypeptid, umfassend eine variable Domäne der schweren Kette eines Antikörpers, eine Antikörper-CH3-Domäne als Multimerisierungsdomäne und eine variable Domäne der leichten Kette eines Antikörpers,
wobei
- die variable Domäne der schweren Kette des Antikörpers über einen ersten (Peptid-) Linker an einen Terminus der Antikörper-CH3-Multimerisierungsdomäne fusioniert ist,
- die variable Domäne der leichten Kette des Antikörpers über einen zweiten (Peptid-) Linker an den jeweils anderen Terminus der Antikörper-CH3-Multimerisierungsdomäne fusioniert ist und
- die variable Domäne der schweren Kette des Antikörpers eine Intrapeptiddisulfidbindung an die Antikörper-CH3-Multimerisierungsdomäne aufweist,
wobei die variable Domäne der schweren Kette an Position 82b, gemäß Kabat,
einen Cysteinaminosäurerest umfasst,
wobei die Antikörper-CH3-Domäne an Position 433, Nummerierung gemäß Kabat-EU-Index, einen Cysteinaminosäurerest aufweist,
wobei sich die Disulfidbindung zwischen dem Cysteinrest an Position 82b der variablen Domäne der schweren Kette und dem Cysteinrest an Position 433 der CH3-Domäne befindet und
wobei die N-terminale und C-terminale Domäne des Fusionspolypeptids eine Bindungsstelle bilden.

2. Fusionspolypeptid nach Anspruch 1, wobei das Fusionspolypeptid in N- zu C-terminaler Richtung eine variable Domäne der schweren Kette eines Antikörpers, eine Antikörper-CH3-Multimerisieurngsdomäne und eine variable Domäne der leichten Kette eines Antikörpers umfasst.

3. Fusionspolypeptid nach Anspruch 1, wobei das Fusionspolypeptid in N- zu C-terminaler Richtung eine variable Domäne der leichten Kette eines Antikörpers, eine Antikörper-CH3-Multimerisierungsdomäne und eine variable Domäne der schweren Kette eines Antikörpers umfasst.

4. Fusionspolypeptid nach einem der Ansprüche 1 bis 3, wobei der erste und der zweite Peptid-Linker die Aminosäuresequenz SEQ ID NO:01 und SEQ ID NO:02 aufweisen.

5. Fusionspolypeptid nach einem der Ansprüche 1 bis 3, wobei der erste und der zweite Peptid-Linker die Aminosäuresequenz SEQ ID NO:03 und SEQ ID NO:04 aufweisen.

6. Fusionspolypeptid nach einem der Ansprüche 1 bis 3, wobei der erste und der zweite Peptid-Linker die Aminosäuresequenz SEQ ID NO:05 und SEQ ID NO:06 aufweisen.

7. Fusionspolypeptid nach einem der Ansprüche 1 bis 3, wobei der erste und der zweite Peptid-Linker die Aminosäuresequenz SEQ ID NO:07 und SEQ ID NO:08 aufweisen.

8. Antikörper, bestehend aus zwei Fusionspolypeptiden nach einem der Ansprüche 1 bis 7.

9. Antikörper nach Anspruch 8, wobei
- eine der CH3-Domänen die Mutation T366W enthält und die andere der CH3-Domänen die Mutationen T366S, L368A und Y407V enthält (Nummerierung gemäß dem Kabat-EU-Index)
und
- wobei eine der CH3-Domänen zusätzlich die Mutation Y349C enthält und die andere der CH3-Domänen zusätzlich die Mutation E356C oder S354C enthält (Nummerierung gemäß dem Kabat-EU-Index).

10. Antikörper nach einem der Ansprüche 8 bis 9, wobei der Antikörper ein bispezifischer Antikörper ist.

11. Antikörper nach einem der Ansprüche 8 bis 10 zur Verwendung als ein Medikament.

12. Antikörper nach einem der Ansprüche 8 bis 10 zur Verwendung bei der Behandlung einer Augengefäßkrankheit.

13. Antikörper zur Verwendung nach Anspruch 12, wobei die Augengefäßkrankheit altersbedingte Makuladegeneration oder diabetische Retinopathie ist.

14. Pharmazeutische Formulierung, enthaltend das Fusionspolypeptid nach einem der Ansprüche 1 bis 7 oder den Antikörper nach einem der Ansprüche 8 bis 10.

## Revendications

1. Polypeptide de fusion comprenant un domaine variable de chaîne lourde d'anticorps, un domaine CH3 d'anticorps en tant que domaine de multimérisation et un domaine variable de chaîne légère d'anticorps,
dans lequel
- le domaine variable de chaîne lourde d'anticorps est fusionné via un premier lieur (peptidique) à une extrémité du domaine de multimérisation CH3 d'anticorps,
- le domaine variable de chaîne légère d'anticorps est fusionné via un second lieur (peptidique) à l'autre extrémité respective du domaine de multimérisation CH3 d'anticorps, et
- le domaine variable de chaîne lourde d'anticorps a une liaison disulfure intrapeptidique avec le domaine de multimérisation CH3 d'anticorps,
dans lequel le domaine variable de chaîne lourde comprend au niveau de la position 82b, selon Kabat,
un résidu d'acide aminé cystéine,
dans lequel le domaine CH3 d'anticorps a au niveau de la position 433, numérotation selon l'indice EU de Kabat, un résidu d'acide aminé cystéine,
dans lequel la liaison disulfure est entre le résidu cystéine au niveau de la position 82b du domaine variable de chaîne lourde et le résidu cystéine au niveau de la position 433 du domaine CH3, et
dans lequel les domaines N-terminal et C-terminal du polypeptide de fusion forment un site de liaison.

2. Polypeptide de fusion selon la revendication 1, dans lequel le polypeptide de fusion comprend dans la direction N- vers C-terminale un domaine variable de chaîne lourde d'anticorps, un domaine de multimérisation CH3 d'anticorps et un domaine variable de chaîne légère d'anticorps.

3. Polypeptide de fusion selon la revendication 1, dans lequel le polypeptide de fusion comprend dans la direction N- vers C-terminale un domaine variable de chaîne légère d'anticorps, un domaine de multimérisation CH3 d'anticorps et un domaine variable de chaîne lourde d'anticorps.

4. Polypeptide de fusion selon l'une quelconque des revendications 1 à 3, dans lequel le premier et le second lieurs peptidiques ont la séquence d'acides aminés SEQ ID NO : 01 et SEQ ID NO : 02.

5. Polypeptide de fusion selon l'une quelconque des revendications 1 à 3, dans lequel le premier et le second lieurs peptidiques ont la séquence d'acides aminés SEQ ID NO : 03 et SEQ ID NO : 04.

6. Polypeptide de fusion selon l'une quelconque des revendications 1 à 3, dans lequel le premier et le second lieurs peptidiques ont la séquence d'acides aminés SEQ ID NO : 05 et SEQ ID NO : 06.

7. Polypeptide de fusion selon l'une quelconque des revendications 1 à 3, dans lequel le premier et le second lieurs peptidiques ont la séquence d'acides aminés SEQ ID NO : 07 et SEQ ID NO : 08.

8. Anticorps constitué de deux polypeptides de fusion selon l'une quelconque des revendications 1 à 7.

9. Anticorps selon la revendication 8, dans lequel
- l'un des domaines CH3 contient la mutation T366W et l'autre des domaines CH3 contient les mutations T366S, L368A et Y407V (numérotation selon l'indice EU de Kabat),
et
- dans lequel l'un des domaines CH3 contient de plus la mutation Y349C et l'autre des domaines CH3 contient de plus la mutation E356C ou S354C (numérotation selon l'indice EU de Kabat).

10. Anticorps selon l'une quelconque des revendications 8 à 9, dans lequel l'anticorps est un anticorps bispécifique.

11. Anticorps selon l'une quelconque des revendications 8 à 10 pour une utilisation comme médicament.

12. Anticorps selon l'une quelconque des revendications 8 à 10 pour une utilisation dans le traitement d'une maladie vasculaire oculaire.

13. Anticorps pour une utilisation selon la revendication 12, dans lequel la maladie vasculaire oculaire est une dégénérescence maculaire liée à l'âge ou une rétinopathie diabétique.

14. Formulation pharmaceutique contenant le polypeptide de fusion selon l'une quelconque des revendications 1 à 7 ou l'anticorps selon l'une quelconque des revendications 8 à 10.
